# EUROPEAN PATENT APPLICATION

(11) **EP 4 670 618 A1**
(43) Date of publication of application: **31.12.2025**
(21) Application number: 23930839.8
(22) Date of filing: 17.11.2023
(51) Int. Cl.: A61B 3/15

(54) **OPHTHALMIC DEVICE AND METHOD FOR OPERATING OPHTHALMIC DEVICE**

(30) Priority: 28.03.2023 JP 2023052057
(71) Applicant: Topcon Corporation, Tokyo 174-8580 (JP)
(72) Inventor: MOCHIZUKI Yuki, Tokyo 174-8580 (JP); SHIBANO Takaaki, Tokyo 174-8580 (JP); SUZUKI Minami, Tokyo 174-8580 (JP); YAMABE Masaru, Tokyo 174-8580 (JP); TSUKIHARA Kouichi, Tokyo 174-8580 (JP)
(74) Representative: Schmitt-Nilson Schraud Waibel Wohlfrom Patentanwälte Partnerschaft mbB
(86) International application number: PCT/JP2023/041452
(87) International publication number: WO 2024/202212

(57) **Abstract**

There are provided an ophthalmic device capable of reliably avoiding an examination head coming closer to a nose of a subject and a method for operating the ophthalmic device. The ophthalmic device includes a displacement mechanism (an XZ movement mechanism (16), a Y movement mechanism (18), a swing rotation mechanism (20), and a tilt rotation mechanism (80)) configured to displace an examination head (22) with respect to a subject eye (E), a drive controlling unit (46) configured to drive the displacement mechanism to displace the examination head (22) to an examination position for the subject eye (E) along an axis (TA) of tilt, a distance detecting unit (a stereo camera (34) and a detection controlling unit (47A)) configured to detect a face distance (Fd) which is a distance between the examination head (22) and a face of a subject while the examination head (22) is displaced along the axis (TA) of tilt by the displacement mechanism, and a retraction controlling unit (47B) configured to drive the displacement mechanism to rotate the examination head (22) in a direction of increasing the face distance (Fd) around a rotation axis determined in advance when the face distance (Fd) detected by the distance detecting unit is less than a threshold determined in advance.

## Description

### {Technical Field}

The presently disclosed subject matter relates to an ophthalmic device which aligns an examination head with a subject eye and a method for operating the ophthalmic device.

### {Background Art}

In ophthalmology, ophthalmic examinations (e.g., acquisition of various ocular characteristics, such as ocular refractive power, an intraocular pressure, and the number of corneal endothelial cells, of a subject eye, fundus imaging, and tomography) on the subject eye are performed by an ophthalmic device. Alignment of an examination head (also referred to as an examination unit) of the ophthalmic device with respect to the subject eye is extremely important in terms of, e.g., accuracy, reliability, and image quality of a result of the examinations on the subject eye.

For this reason, each of the ophthalmic devices according to Patent Literatures 1 and 2 stereoscopically photographs a subject eye using a stereo camera and executes alignment detection that detects a relative position of the subject eye to an examination head based on anterior eye part images of the subject eye obtained by the stereoscopic photographing. The ophthalmic device moves the examination head through electromotive driving based on a result of the alignment detection, thereby executing automatic alignment of the examination head with the subject eye.

### {Citation List}

### {Patent Literature}

{Patent Literature 1} Japanese Patent Application Laid-Open No. 2013-248376
{Patent Literature 2} Japanese Patent Application Laid-Open No. 2021-069415

### {Summary of Invention}

### {Technical Problem}

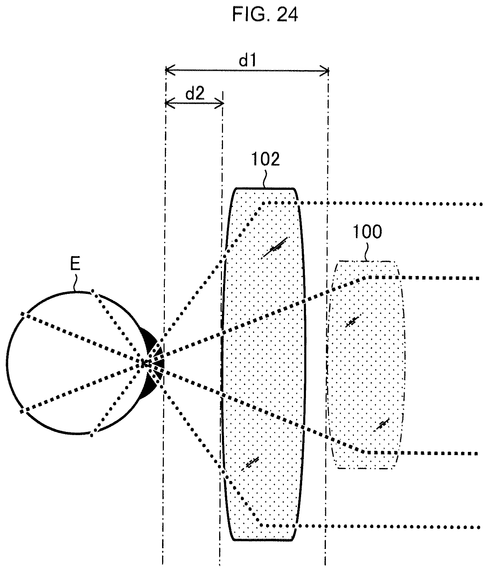

Figure 24 is an explanatory diagram for explaining a relationship between a lens size of an objective lens in an examination head and an operating distance of the examination head. As illustrated in Figure 24, a photographic angle of view of a conventional objective lens 100 in an examination head is about 45°. An ophthalmic device such as a fundus camera, an Optical Coherence Tomography (OCT) device, or a Scanning Laser Ophthalmoscope (SLO) device supporting wide-angle photographing has an objective lens 102 larger than the objective lens 100 in an examination head due to optical constraints. As a result, an operating distance d2 between a subject eye E and the examination head with the objective lens 102 is shorter than an operating distance d1 with the objective lens 100.

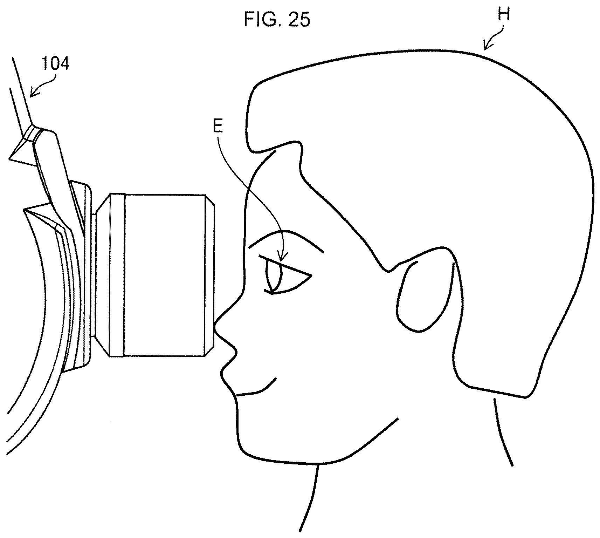

Figure 25 is an explanatory view for explaining a problem arising from a short operating distance between the subject eye E and an examination head 104. For example, when an objective lens diameter of the examination head 104 is 80 mm, and an operating distance of the examination head 104 is 50 mm, as illustrated in Figure 25, a distance between a forehead of the subject H and the examination head 104 is about 22 mm, a distance between a nose of the subject H and the examination head 104 is about 2 mm, and a distance between cheeks of the subject H and the examination head 104 is about 28 mm. The distance between the nose of the subject H and the examination head 104 is thus particularly short. As a result, in executing automatic alignment of the examination head 104 with the subject eye E using the methods according to Patent Literatures 1 and 2 described above, the examination head 104 needs to be brought close to the nose of the subject H. Additionally, the examination head 104 has an increased likelihood of coming close to the nose of the subject H depending on the shape and size of the nose of the subject H.

The presently disclosed subject matter has been made in view of the above-described circumstances, and has as its object to provide an ophthalmic device capable of reliably avoiding an examination head coming close to a subject's nose and a method for operating the ophthalmic device.

### {Solution to Problem}

An ophthalmic device for achieving the object of the presently disclosed subject matter includes: an examination head configured to examine a subject eye; a displacement mechanism configured to displace the examination head with respect to the subject eye; a drive controlling unit configured to drive the displacement mechanism to displace the examination head to an examination position for the subject eye along an axis of tilt, wherein the axis of tilt is an axis obtained by tilting, around the subject eye, a reference axis that extends along an eye direction of the subject eye in parallel with a front-back direction serving as an operating distance direction of the examination head, outward away from a subject's nose; a distance detecting unit configured to detect a face distance which is a distance between the examination head and a face of the subject while the examination head is displaced along the axis of tilt by the displacement mechanism; and a retraction controlling unit configured to drive the displacement mechanism to rotate the examination head in a direction of increasing the face distance around a rotation axis determined in advance when the face distance detected by the distance detecting unit is less than a threshold determined in advance.

According to the ophthalmic device, it is possible to avoid the examination head coming close to the face of the subject by driving a rotation mechanism to rotate the examination head when the face distance detected by the distance detecting unit is less than the threshold determined in advance.

In the ophthalmic device according to another aspect of the presently disclosed subject matter, the distance detecting unit detects, as the face distance, a distance between the examination head and the subject's nose. This makes it possible to avoid the examination head coming close to the subject's nose.

In the ophthalmic device according to another aspect of the presently disclosed subject matter, the displacement mechanism includes a movement mechanism configured to move the examination head in the front-back direction, a left-right direction, and an up-down direction with respect to the subject eye, and a rotation mechanism configured to rotate the examination head around the rotation axis, and the retraction controlling unit executes first retraction control that drives the rotation mechanism to rotate the examination head in the direction of increasing the face distance and second retraction control that stops driving of the movement mechanism or drives the movement mechanism to retract the examination head in a direction away from the face. This makes it possible to avoid the examination head coming close to the face of the subject.

In the ophthalmic device according to another aspect of the presently disclosed subject matter, the displacement mechanism includes a movement mechanism configured to move the examination head in the front-back direction, a left-right direction, and an up-down direction with respect to the subject eye, and a rotation mechanism configured to rotate the examination head around the rotation axis, the examination head includes an objective lens, the rotation mechanism rotates the examination head around the objective lens, and the movement mechanism integrally moves the examination head and the rotation axis in the front-back direction, the left-right direction, and the up-down direction.

In the ophthalmic device according to another aspect of the presently disclosed subject matter, the rotation axis is parallel to the up-down direction, and the outward direction is parallel to the left-right direction.

In the ophthalmic device according to another aspect of the presently disclosed subject matter, the rotation axis is perpendicular to the up-down direction, and the outward direction is an upward direction of the up-down direction.

In the ophthalmic device according to another aspect of the presently disclosed subject matter, the displacement mechanism includes a movement mechanism configured to move the examination head in the front-back direction, a left-right direction, and an up-down direction with respect to the subject eye, and a rotation mechanism configured to rotate the examination head around the rotation axis, the examination head includes an objective lens, the rotation axis is located on a front side in the front-back direction which is closer to the subject eye than the objective lens is, and the movement mechanism integrally moves the examination head and the rotation axis in the front-back direction, the left-right direction, and the up-down direction.

In the ophthalmic device according to another aspect of the presently disclosed subject matter, the rotation axis is parallel to the up-down direction, and the outward direction is parallel to the left-right direction.

In the ophthalmic device according to another aspect of the presently disclosed subject matter, the rotation axis is perpendicular to the up-down direction, and the outward direction is an upward direction of the up-down direction.

In the ophthalmic device according to another aspect of the presently disclosed subject matter, the distance detecting unit includes a plurality of cameras provided at the examination head, and a detection controlling unit configured to continuously execute photographing control that causes the plurality of cameras to photograph the face from a plurality of directions different from each other and computation of the face distance based on respective photographed images of the face photographed by the plurality of cameras while the examination head is displaced along the axis of tilt by the displacement mechanism. This makes it possible to continuously detect the face distance while the examination head is displaced along the axis of tilt by the displacement mechanism.

In the ophthalmic device according to another aspect of the presently disclosed subject matter, the distance detecting unit includes a plurality of cameras provided at the examination head, a prior photographing controlling unit configured to cause one particular camera capable of photographing the nose among the plurality of cameras to photograph the nose at an initial position for the examination head, and a detection controlling unit configured to continuously execute photographing control that causes the particular camera to photograph the nose and a computation process of comparing a photographed image of the nose newly photographed by the particular camera with a photographed image of the nose photographed at the initial position to compute the face distance while the examination head is displaced along the axis of tilt by the displacement mechanism. This makes it possible to continuously detect the face distance even if the nose is photographable by only one camera (the particular camera) while the examination head is displaced along the axis of tilt by the displacement mechanism.

In the ophthalmic device according to another aspect of the presently disclosed subject matter, the distance detecting unit includes a non-contact distance sensor provided at the examination head, and a detection controlling unit configured to continuously execute computation of the face distance based on a detection signal output from the distance sensor while the examination head is displaced along the axis of tilt by the displacement mechanism. This makes it possible to continuously detect the face distance while the examination head is displaced along the axis of tilt by the displacement mechanism.

A method for operating an ophthalmic device for achieving the object of the presently disclosed subject matter is a method for operating an ophthalmic device including an examination head configured to examine a subject eye and a displacement mechanism configured to displace the examination head with respect to the subject eye, the method including: a drive controlling step of driving the displacement mechanism to displace the examination head to an examination position for the subject eye along an axis of tilt, wherein the axis of tilt is an axis obtained by tilting, around the subject eye, a reference axis that extends along an eye direction of the subject eye in parallel with a front-back direction serving as an operating distance direction of the examination head, outward away from a subject's nose; a distance detecting step of detecting a face distance which is a distance between the examination head and a face of the subject while the examination head is displaced along the axis of tilt by the displacement mechanism; and a retraction controlling step of driving the displacement mechanism to rotate the examination head in a direction of increasing the face distance around a rotation axis determined in advance when the face distance detected in the distance detecting step is less than a threshold determined in advance.

### {Advantageous Effects of Invention}

According to the presently disclosed subject matter, it is possible to reliably avoid an examination head coming close to a subject's nose.

### {Brief Description of Drawings}

Figure 1 is a side view of an ophthalmic device according to a first embodiment;
Figure 2 is a front view of a lens-barrel as viewed from a front side in a Z direction;
Figure 3 is a cross-sectional view of the lens-barrel taken along line 3-3 in Figure 2;
Figure 4 is a block diagram illustrating a configuration of the ophthalmic device according to the first embodiment;
Figure 5 is an explanatory diagram for explaining a method for automatic alignment of an examination head;
Figure 6 is an explanatory diagram for explaining an example 1-1 of the automatic alignment of the examination head according to the first embodiment;
Figure 7 is an explanatory diagram for explaining an example 1-2 of the automatic alignment of the examination head according to the first embodiment;
Figure 8 is an explanatory diagram for explaining an example 1-3 of the automatic alignment of the examination head according to the first embodiment;
Figure 9 is an explanatory diagram for explaining the likelihood of the examination head coming close to a face of a subject during the automatic alignment;
Figure 10 is an explanatory diagram for explaining retraction control of the examination head according to the first embodiment;
Figure 11 is an explanatory diagram for explaining a modification of the retraction control of the examination head according to the first embodiment;
Figure 12 is a flowchart illustrating a flow of a process of examining a subject eye by the ophthalmic device according to the first embodiment;
Figure 13 is a flowchart illustrating a flow of an automatic alignment process for the examination head;
Figure 14 is an explanatory diagram for explaining displacement of the examination head after the start of the automatic alignment;
Figure 15 is a side view of an ophthalmic device according to a second embodiment;
Figure 16 is an explanatory diagram for explaining an example 2-1 of automatic alignment of an examination head according to the second embodiment;
Figure 17 is an explanatory diagram for explaining an example 2-2 of the automatic alignment of the examination head according to the second embodiment;
Figure 18 is an explanatory diagram for explaining retraction control of the examination head according to the second embodiment;
Figure 19 is a front view of a lens-barrel distal end face of an ophthalmic device according to a third embodiment;
Figure 20 is a side view of an ophthalmic device according to a fourth embodiment;
Figure 21 is an explanatory diagram for explaining an example 3 of automatic alignment of an examination head according to the fourth embodiment;
Figure 22 is a side view of an ophthalmic device according to a fifth embodiment;
Figure 23 is an explanatory diagram for explaining an example 4 of automatic alignment of an examination head according to the fifth embodiment.
Figure 24 is an explanatory diagram for explaining a relationship between a lens size of an objective lens in an examination head and an operating distance of the examination head; and
Figure 25 is an explanatory view for explaining a problem arising from a short operating distance between a subject eye and an examination head.

### {Description of Embodiments}

### [First Embodiment]

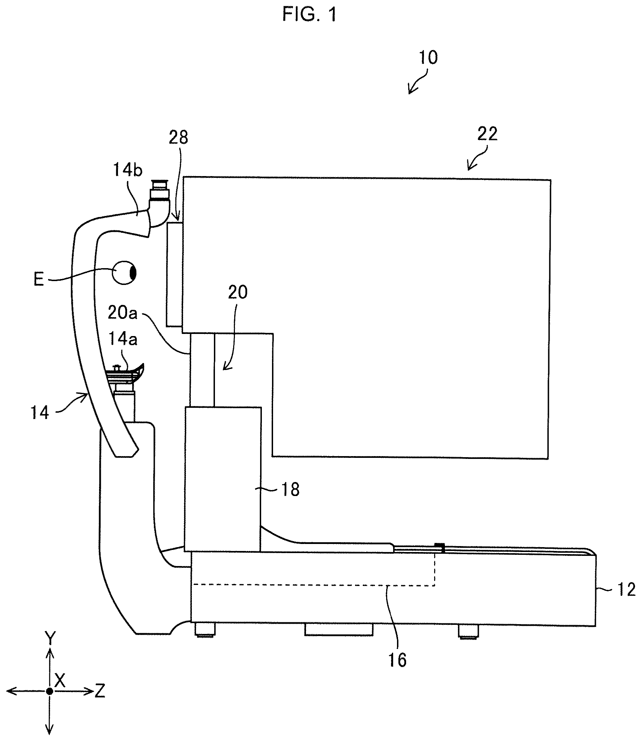

Figure 1 is a side view of an ophthalmic device 10 according to a first embodiment. An X direction in Figure 1 is a left-right direction based on a subject, a Y direction is an up-down direction, and that a Z direction is a front-back direction (also referred to as an operating distance direction) parallel to a forward direction toward the subject (a subject eye E) and a rearward direction away from the subject.

As illustrated in Figure 1, the ophthalmic device 10 is a multifunction machine which is a combination of a fundus camera that executes fundus imaging of the subject eye E and an optical coherence tomograph that obtains a tomographic image of the subject eye E using OCT. The ophthalmic device 10 includes a base 12, a face support 14, an XZ movement mechanism 16, a Y movement mechanism 18, a swing rotation mechanism 20, and an examination head 22.

The face support 14 is attached to a front end portion on a front side (a subject eye E side) in the Z direction of the base 12. The XZ movement mechanism 16 is also provided at the base 12.

The face support 14 includes a chin rest 14a and a forehead rest 14b which are positionally adjustable in the Y direction (up-down direction), and supports a face of the subject at a position facing the examination head 22 (a lens-barrel 28).

The XZ movement mechanism 16 together with the Y movement mechanism 18 (to be described later) constitutes a movement mechanism according to the presently disclosed subject matter. The XZ movement mechanism 16 includes a pedestal which is movable in each of the X and Z directions with respect to the base 12 and an electric drive mechanism (a publicly known actuator, such as a motor drive mechanism) which moves the pedestal in each of the X and Z directions (both not illustrated). The XZ movement mechanism 16 integrally moves the Y movement mechanism 18, the swing rotation mechanism 20, and the examination head 22 in the X and Z directions.

The Y movement mechanism 18 includes a lifting table which is movable in the Y direction and an electric drive mechanism which moves the lifting table in the Y direction (both not illustrated). The Y movement mechanism 18 integrally moves the swing rotation mechanism 20 and the examination head 22 in the Y direction. With this configuration, the XZ movement mechanism 16 and the Y movement mechanism 18 can integrally move the swing rotation mechanism 20 and the examination head 22 in the X, Y, and Z directions.

The swing rotation mechanism 20 corresponds to a rotation mechanism according to the presently disclosed subject matter and, together with the XZ movement mechanism 16 and the Y movement mechanism 18 described earlier, constitutes a displacement mechanism according to the presently disclosed subject matter. The swing rotation mechanism 20 includes a rotating shaft 20a parallel to the Y direction and an electric drive mechanism which rotates the rotating shaft 20a, and rotates (swings) the examination head 22 around the rotating shaft 20a.

The examination head 22 is attached to an upper end portion in the Y direction of the rotating shaft 20a. With this configuration, the examination head 22 is movable in the X, Y, and Z direction by the XZ movement mechanism 16 and the Y movement mechanism 18, and is rotatable in a direction around the rotating shaft 20a by the swing rotation mechanism 20.

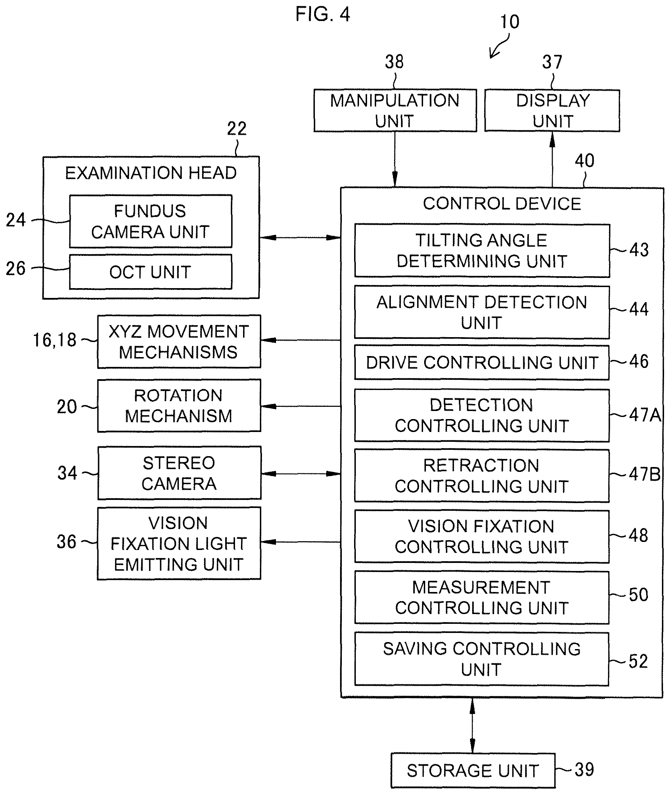

The examination head 22 includes a fundus camera unit 24 and an OCT unit 26 (to be described later) illustrated in Figure 4, and the lens-barrel 28. The fundus camera unit 24 photographs a fundus of the subject eye E through an objective lens 30 (to be described later) and outputs a fundus image which is a frontal image of the fundus to a control device 40 (to be described later) (see Figure 4). The OCT unit 26 performs OCT imaging of the subject eye E through the objective lens 30 and outputs a signal such as a detection signal needed to generate a tomographic image of the subject eye E to the control device 40. Specific configurations of the fundus camera unit 24 and the OCT unit 26 are publicly known techniques (see Patent Literature 1 described above) and that a specific description thereof will be omitted.

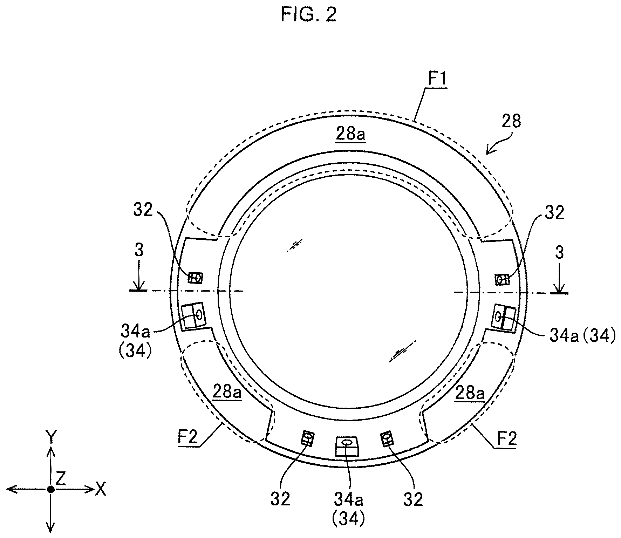

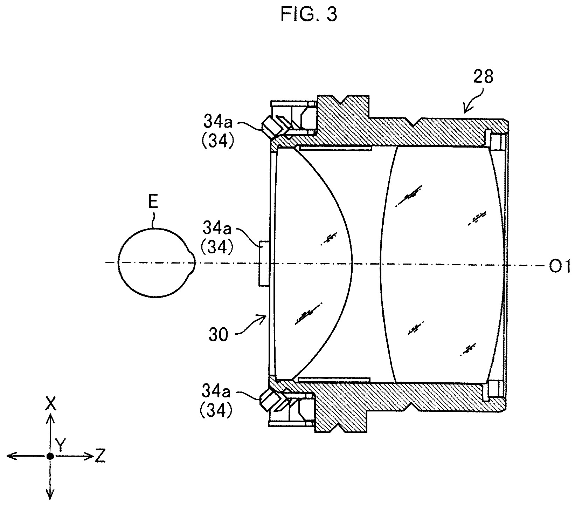

Figure 2 is a front view of the lens-barrel 28 as viewed from the front side in the Z direction. Figure 3 is a cross-sectional view of the lens-barrel 28 taken along line 3-3 in Figure 2. Figures 1 to 3 illustrates an example which provides the lens-barrel 28 at an end portion on the front side in the Z direction of the examination head 22. The lens-barrel 28 houses (holds) the objective lens 30 having an optical axis O1 (see Figure 3) parallel to the Z direction. Four illumination light sources 32 and a stereo camera 34 are provided at a lens-barrel distal end face 28a on the front side in the Z direction of the lens-barrel 28. As the objective lens 30, for example, a large lens supporting wide-angle photographing, i.e., a lens with a short operating distance is used (see Figure 24). The type of the objective lens 30 is not particularly limited and that a lens with a photographic angle of view of about 45° may be used.

When the objective lens 30 and the rotating shaft 20a described earlier are viewed from a one-direction side in the Y direction, a position of the rotating shaft 20a and a position of the objective lens 30 coincide (the term "coincide" as used herein is intended to include the meaning of "substantially coincide"; the same applies hereinafter). With this configuration, the examination head 22 is rotated (swung) around the objective lens 30 by the swing rotation mechanism 20.

Respective illumination light sources 32 are provided at two end portions in the X direction (two left and right end portions) of the lens-barrel distal end face 28a, and two illumination light sources 32 are provided at a lower end portion of the lens-barrel distal end face 28a such that a camera 34a (to be described later) is sandwiched therebetween. Each illumination light source 32 is, for example, a Light Emitting Diode (LED) light source and illuminates the subject eye E.

The stereo camera 34 is used for alignment detection that detects relative positions in the X, Y, and Z directions of the subject eye E to the examination head 22. The stereo camera 34 includes a plurality of cameras 34a. For example, in the present embodiment, the stereo camera 34 includes two cameras 34a provided at the two end portions in the X direction (the two left and right end portions) corresponding to positions on the left and right of the objective lens 30 in the lens-barrel distal end face 28a and one camera 34a provided at the lower end portion corresponding to a position below the objective lens 30 in the lens-barrel distal end face 28a, three cameras 34a in total. The cameras 34a simultaneously photograph an anterior eye part of the subject eye E from a plurality of (three in the present embodiment) directions different from each other at the time of the alignment detection and output a plurality of (three) anterior eye part images of the subject eye E to the control device 40 (see Figure 4). The number of cameras 34a may be two, or four or more.

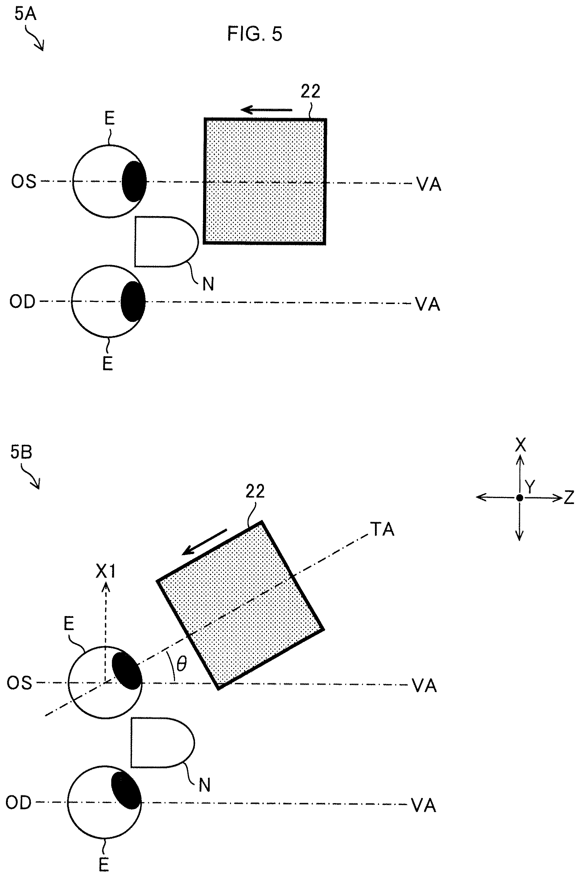

Positions of the cameras 34a may be appropriately changed. For example, if the camera 34a is provided in an upper region F1 (see Figure 2) above (in the Y direction) the two end portions in the X direction in the lens-barrel distal end face 28a, a pupil of the subject eye E may be hidden by upper lashes of the subject in photographing the subject eye E by the camera 34a. Additionally, if respective cameras 34a are provided in left and right lower oblique regions F2 (see Figure 2) between the two end portions in the X direction and the lower end portion in the lens-barrel distal end face 28a, each camera 34a is likely to come close to a nose N (see Figure 5) of the subject during alignment of the examination head 22. For this reason, the cameras 34a are preferably provided at the two end portions in the X direction and the lower end portion of the lens-barrel distal end face 28a.

Figure 4 is a block diagram illustrating a configuration of the ophthalmic device 10 according to the first embodiment. As illustrated in Figure 4, the ophthalmic device 10 includes a vision fixation light emitting unit 36, a display unit 37, a manipulation unit 38, a storage unit 39, and the control device 40 in addition to the XZ movement mechanism 16, the Y movement mechanism 18, the swing rotation mechanism 20, the examination head 22, and the stereo camera 34 described earlier.

The vision fixation light emitting unit 36 guides and fixes an eye direction of the subject eye E by emitting vision fixation light (a bright spot image) toward the subject eye E. The vision fixation light emitting unit 36 includes a publicly known vision fixation target display unit, a plurality of vision fixation holes, and an external fixation lamp (all not illustrated) (see Patent Literature 2 described above).

The vision fixation target display unit is provided inside the examination head 22 and is used for internal vision fixation that projects vision fixation light (e.g., a bright spot image) onto the subject eye E through the objective lens 30. The vision fixation holes are provided at a front surface in the Z direction (which may be the lens-barrel distal end face 28a) of the examination head 22 so as to surround the objective lens 30 and are used for peripheral vision fixation. The peripheral vision fixation is a vision fixation method for selectively lighting the vision fixation holes, thereby causing the subject eye E to make a great circumnutation in a desired direction. The external fixation lamp is provided at the face support 14 or the examination head 22 and is used for external vision fixation. The external vision fixation is a vision fixation method for causing the subject eye E to make a circumnutation in an arbitrary direction or make a greater circumnutation than under internal vision fixation by adjusting a light source position of the external fixation lamp, or adjusting an orientation of the subject eye E by guiding a visual line of the subject eye E or a fellow eye when the internal vision fixation cannot be performed.

As the display unit 37, which is a type of display device, for example, a touch-panel monitor is used. The display unit 37 displays screens such as a setup screen for the ophthalmic device 10, a manipulation screen (User Interface (UI) screen) for the ophthalmic device 10, anterior eye part images of the subject eye E photographed by the stereo camera 34, a result (a fundus image and a tomographic image of the subject eye E) of examining the subject eye E by the examination head 22.

Examples of the manipulation unit 38 include a publicly known manipulation lever, switches and a manipulation screen to be displayed on the display unit 37 (all not illustrated). The manipulation unit 38 is used to input an instruction such as a positional adjustment of the chin rest 14a and the forehead rest 14b, an XYZ movement and a rotation manipulation of the examination head 22, selecting the type of examination (from fundus imaging and OCT imaging), switching between automatic alignment and manual alignment, an examination start or saving an examination result (a fundus image or a tomographic image).

The storage unit 39 is a recording medium (storage medium) which stores a program to be executed by the control device 40, and various publicly known storages are used as the storage unit 39. A fundus image of the subject eye E photographed by the fundus camera unit 24 and a tomographic image of the subject eye E obtained through OCT imaging by the OCT unit 26 are saved in the storage unit 39.

The control device 40 performs overall control on the action of the units of the ophthalmic device 10 and executes the control such as alignment of the examination head 22 with the subject eye E, imaging of the fundus of the subject eye E by the fundus camera unit 24, and OCT imaging of the subject eye E by the OCT unit 26.

The control device 40 includes an arithmetic circuit including various processors and memories. Examples of the various processors include a Central Processing Unit (CPU), a Graphics Processing Unit (GPU), an Application Specific Integrated Circuit (ASIC), programmable logic devices (e.g., a Simple Programmable Logic Devices (SPLD), a Complex Programmable Logic Device (CPLD), and a Field Programmable Gate Arrays (FPGA)). Various functions of the control device 40 may be implemented by one processor or may be implemented by a plurality of processors of the same type or of different types.

The control device 40 functions as a tilting angle determining unit 43, an alignment detection unit 44, a drive controlling unit 46, a detection controlling unit 47A, a retraction controlling unit 47B, a vision fixation controlling unit 48, a measurement controlling unit 50, and a saving controlling unit 52 by executing a control program stored in the storage unit 39.

Figure 5 is an explanatory diagram for explaining a method for automatic alignment of the examination head 22. Here, reference character "OD" in Figure 5 denotes a right eye (oculus dexter) and that reference character "OS" denotes a left eye (oculus sinister). As described earlier, in the ophthalmic device 10 (the fundus camera and the OCT device) supporting wide-angle photographing, an operating distance of the examination head 22 is short due to the objective lens 30 of large size. For this reason, as indicated by reference character 5A in Figure 5, when the examination head 22 is moved from a position in front of the subject eye E (the left eye OS here) toward the front side in the Z direction at the time of the automatic alignment of the examination head 22, the examination head 22 may come close to the subject's nose N.

For the above-described reason, as indicated by reference character 5B in Figure 5, when viewed from the one-direction side in the Y direction, the ophthalmic device 10 according to the present embodiment brings the examination head 22 closer to the subject eye E from an oblique direction during the automatic alignment of the examination head 22.

Specifically, assume that an axis along the eye direction (an eye direction of the subject eye E observing infinite distance) of the subject eye E parallel to the Z direction is a reference axis VA, that for X direction, the outward direction away from the nose (N), based on the subject eye E (here, the left eye OS), is defined as X1. Furthermore, an axis obtained by tilting the reference axis VA in the outward direction X1 around the subject eye E is designated as an axis TA of tilt. The examination head 22 is displaced to an examination position where examination (fundus imaging and OCT imaging) of the subject eye E is executable (hereinafter simply referred to as the examination position) along the axis TA of tilt when viewed from the one-direction side in the Y direction. The term "displacement" here subsumes movement in the X, Y, and Z directions and rotation of the examination head 22.

The tilting angle determining unit 43 determines a tilting angle θ of the axis TA of tilt with respect to the reference axis VA when viewed from the one-direction side in the Y direction, i.e., the tilting angle θ of the axis TA of tilt with respect to the reference axis VA in an XZ plane. For example, the tilting angle determining unit 43 determines, as the tilting angle θ, a value that the examiner selects from a plurality of angles (e.g., 10°, 15°, and 20°) with the manipulation unit 38 by an examiner and outputs information on the tilting angle θ to the drive controlling unit 46. The tilting angle determining unit 43 may detect a relative position of the nose N to the examination head 22 based on photographed images obtained through stereoscopic photographing of the nose N by the stereo camera 34 and determine the tilting angle θ that can avoid the examination head 22 coming closer to the nose N based on a result of the detection.

Referring back to Figure 4, the alignment detection unit 44 detect a relative position of the subject eye E to the examination head 22 by identification of a pupil center position of the subject eye E and computation of three-dimensional coordinates of the pupil center position based on anterior eye part images of the subject eye E stereoscopically photographed by the cameras 34a of the stereo camera 34 during the automatic alignment of the examination head 22. Since a method for alignment detection using the stereo camera 34 is a publicly known technique (see Patent Literature 1 described above), a specific description thereof will be omitted.

The drive controlling unit 46 drives the XZ movement mechanism 16, the Y movement mechanism 18, and the swing rotation mechanism 20 to execute alignment of the examination head 22 with the subject eye E and switching of the subject eye E as an examination object (left-right eye switching). The alignment of the examination head 22 includes automatic alignment that is performed by automatically driving the XZ movement mechanism 16, the Y movement mechanism 18, and the swing rotation mechanism 20 and manual alignment that drives the XZ movement mechanism 16, the Y movement mechanism 18, and the swing rotation mechanism 20 in accordance with a manipulation input to the manipulation unit 38. The manipulation unit 38 is configured to execute switching between the automatic alignment and the manual alignment.

The drive controlling unit 46 determines the axis TA of tilt corresponding to the tilting angle θ based on the tilting angle θ initially determined by the tilting angle determining unit 43 at the time of the automatic alignment.

For example, the drive controlling unit 46 identifies the reference axis VA based on photographed images obtained through initial stereoscopic photographing of the face (e.g., the subject eye E or the nose N) of the subject by the stereo camera 34. Alternatively, the drive controlling unit 46 estimates the reference axis VA based on positions in the Y direction of the chin rest 14a and the forehead rest 14b and identification information on the left eye OS and the right eye OD that are already known. The drive controlling unit 46 determines, as the axis TA of tilt, an axis obtained by tilting the reference axis VA in the outward direction X1 around the subject eye E by the tilting angle θ.

The drive controlling unit 46 then drives the XZ movement mechanism 16, the Y movement mechanism 18, and the swing rotation mechanism 20 based on the axis TA of tilt to start automatic alignment that automatically displaces the examination head 22 from an initial position when the ophthalmic device 10 is powered on to the examination position.

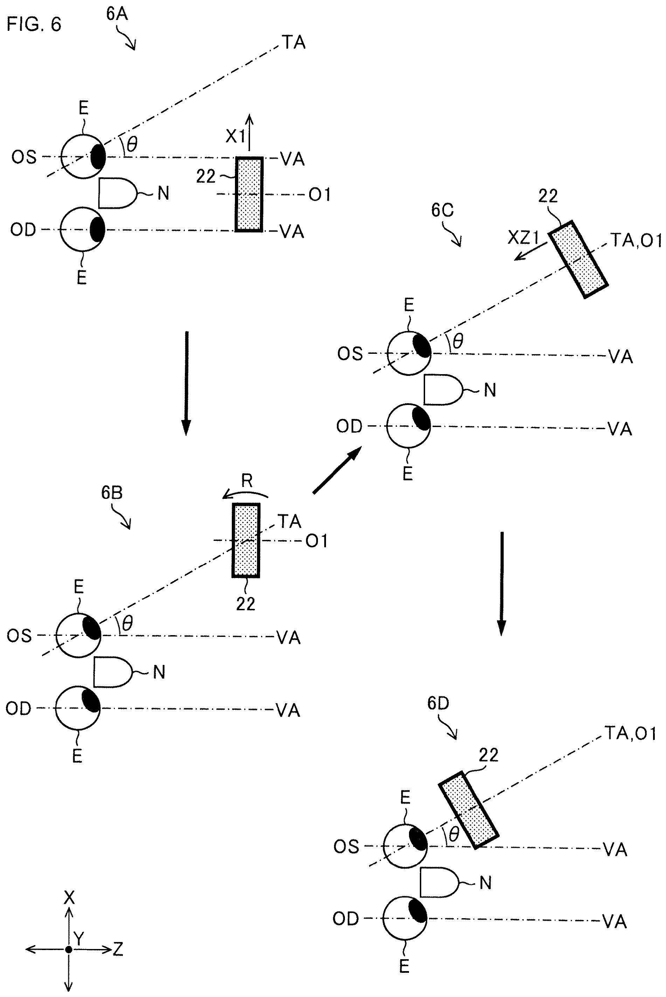

Figure 6 is an explanatory diagram for explaining an example 1-1 of the automatic alignment of the examination head 22 according to the first embodiment. As indicated by reference character 6A in Figure 6, the examination head 22 is initially arranged at a position where the optical axis O1 of the objective lens 30 is between the reference axes VA of the left and right subject eyes E (the left eye OS and the right eye OD) when viewed from the one-direction side in the Y direction, i.e., a position facing (the term "face" as used herein is intended to include the meaning of "substantially face"; the same applies hereinafter) the nose N.

First, the drive controlling unit 46 drives the XZ movement mechanism 16 to execute a first driving process of moving the examination head 22 from the initial position to the axis TA of tilt in the outward direction X1 when the examination head 22 is viewed from the one-direction side in the Y direction.

As indicated by reference character 6B in Figure 6, the drive controlling unit 46 drives the swing rotation mechanism 20 after completion of the first driving process to execute a second driving process of rotating the examination head 22 around the rotating shaft 20a by the tilting angle θ (see an arrow R). With this process, as indicated by reference character 6C in Figure 6, the optical axis O1 of the examination head 22 (the objective lens 30) becomes parallel to the axis TA of tilt. The second driving process may be executed before the first driving process.

The drive controlling unit 46 then drives the XZ movement mechanism 16 after completion of the second driving process to start a third driving process of moving the examination head 22 to the examination position along the axis TA of tilt when the examination head 22 is viewed from the one-direction side in the Y direction (see an arrow XZ1). With this process, the examination head 22 is moved toward the subject eye E while keeping the tilting angle θ constant (the term "constant" as used herein is intended to include the meaning of "substantially constant"; the same applies hereinafter).

Halfway through the automatic alignment, the examination head 22 is displaced to a position where the alignment detection unit 44 can identify the pupil center position of the subject eye E, i.e., a position where the alignment detection is possible. For this reason, an alignment detection result is input from the alignment detection unit 44 to the drive controlling unit 46. Y-direction positional adjustment of the examination head 22 by the Y movement mechanism 18 may be executed before the alignment detection (at any stage from the first driving process to the third driving process) such that the alignment detection by the alignment detection unit 44 is possible.

As indicated by reference character 6D in Figure 6, the drive controlling unit 46 drives the XZ movement mechanism 16 and the Y movement mechanism 18 based on an alignment detection result input from the alignment detection unit 44 to continue the third driving process until the examination head 22 reaches the examination position. The ophthalmic device 10 executes retraction control of the examination head 22 when the examination head 22 is at risk of coming closer to the face (the nose N) of the subject halfway through the third driving process. The details thereof will be described later. In the third driving process that is executed based on the alignment detection result, the Y-direction positional adjustment of the examination head 22 is also executed.

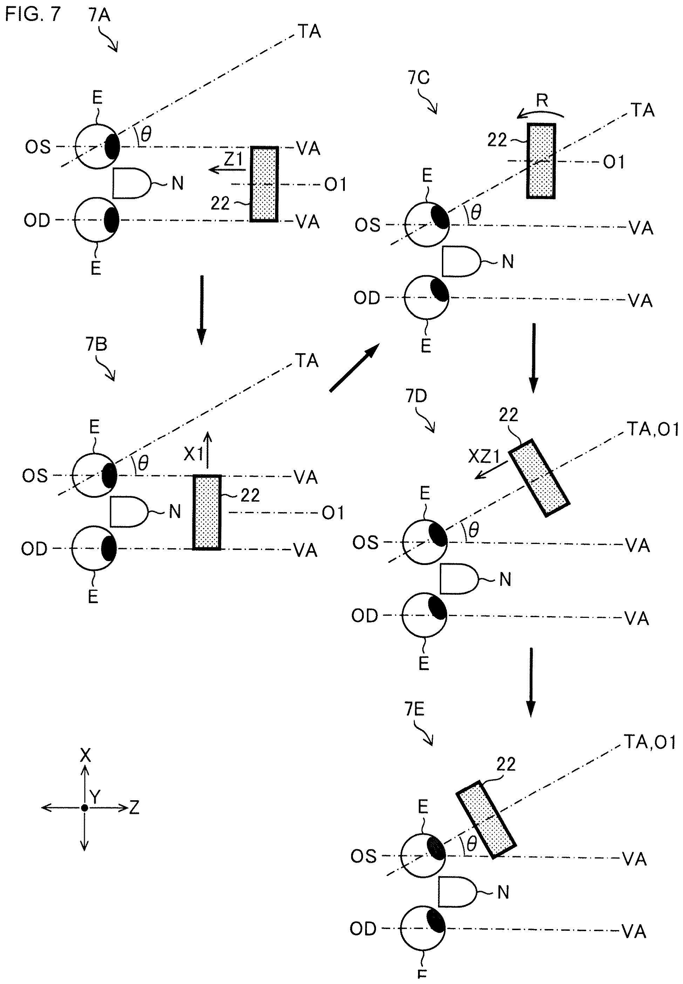

Figure 7 is an explanatory diagram for explaining an example 1-2 of the automatic alignment of the examination head 22 according to the first embodiment. The drive controlling unit 46 drives the XZ movement mechanism 16 to execute a first driving process of first moving the examination head 22 toward the front side (the subject eye E side) in the Z direction by a predetermined distance (see an arrow Z1), as indicated by reference character 7A in Figure 7. The drive controlling unit 46 then moves the examination head 22 to the axis TA of tilt in the outward direction X1, as indicated by reference character 7B in Figure 7. A distance of movement of the examination head 22 toward the front side in the Z direction is not particularly limited as long as a safe distance can be secured between the examination head 22 and the nose N. For example, the distance of movement may be determined based on a result of computing a Z-direction distance from the examination head 22 to the nose N based on photographed images obtained through stereoscopic photographing of the nose N by the stereo camera 34.

Moving the examination head 22 first toward the front side in the Z direction and then in the outward direction X1 to the tilt axis TA can reduce a moving distance of the examination head 22 along the direction X1 compared with the example 1-1 illustrated in Figure 6 described earlier.

As indicated by reference character 7C in Figure 7, the drive controlling unit 46 drives the swing rotation mechanism 20 after completion of the first driving process to execute the same second driving process as the example 1-1 (see reference character 6B in Figure 6) and make the optical axis O1 parallel to the axis TA of tilt. The second driving process may be executed before the first driving process in the example 1-2 as well.

As indicated by reference character 7D in Figure 7, the drive controlling unit 46 drives the XZ movement mechanism 16 after completion of the second driving process to execute a third driving process in the same manner as the example 1-1 (see reference characters 6C and 6D in Figure 6), thereby moving the examination head 22 to the examination position along the axis TA of tilt when the examination head 22 is viewed from the one-direction side in the Y direction. As indicated by reference character 7E in Figure 7, the drive controlling unit 46 drives the XZ movement mechanism 16 and the Y movement mechanism 18 based on alignment detection performed by the alignment detection unit 44 halfway through the automatic alignment to continue the third driving process until the examination head 22 reaches the examination position, unless retraction control (to be described later) is executed.

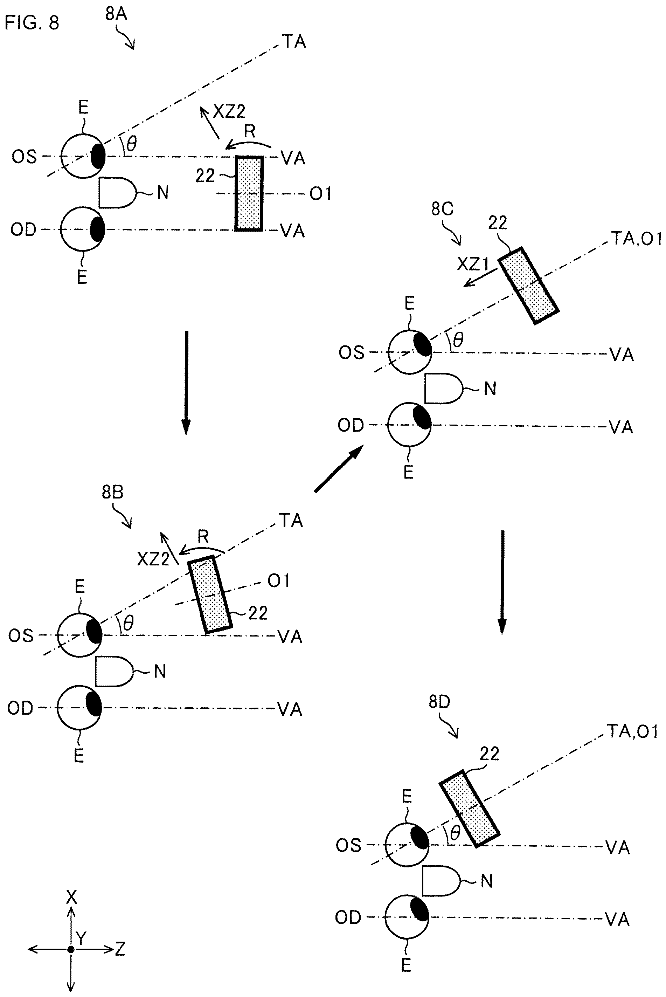

Figure 8 is an explanatory diagram for explaining an example 1-3 of the automatic alignment of the examination head 22 according to the first embodiment. As indicated by reference characters 8A and 8B in Figure 8, the drive controlling unit 46 drives the XZ movement mechanism 16, the Y movement mechanism 18, and the swing rotation mechanism 20 to execute a first driving process of simultaneously executing movement of the examination head 22 toward the front side in the Z direction and in the outward direction X1 and rotation of the examination head 22 by the tilting angle θ (see an arrow XZ2 and the arrow R). With this process, it is possible to move the examination head 22 to the axis TA of tilt in an oblique direction when the examination head 22 is viewed from the one-direction side in the Y direction and make the optical axis O1 parallel to the axis TA of tilt.

In the first driving process described in the example 1-3, the examination head 22 may be displaced to a position on the axis TA of tilt where the stereo camera 34 can photograph the anterior eye part of the subject eye E or to a position where an observation optical system (not illustrated) inside the examination head 22 can photograph the anterior eye part of the subject eye E via a shortest route.

As indicated by reference character 8C in Figure 8, the drive controlling unit 46 drives the XZ movement mechanism 16 and the Y movement mechanism 18 after completion of the first driving process to execute a second driving process. The second driving process in the example 1-3 is the same process as the third driving processes in the example 1-1 and the example 1-2, and moves the examination head 22 to the examination position along the axis TA of tilt when the examination head 22 is viewed from the one-direction side in the Y direction. As indicated by reference character 8D in Figure 8, the drive controlling unit 46 drives the XZ movement mechanism 16 and the Y movement mechanism 18 based on alignment detection performed by the alignment detection unit 44 halfway through the automatic alignment to continue the second driving process until the examination head 22 reaches the examination position, unless the retraction control (to be described later) is executed.

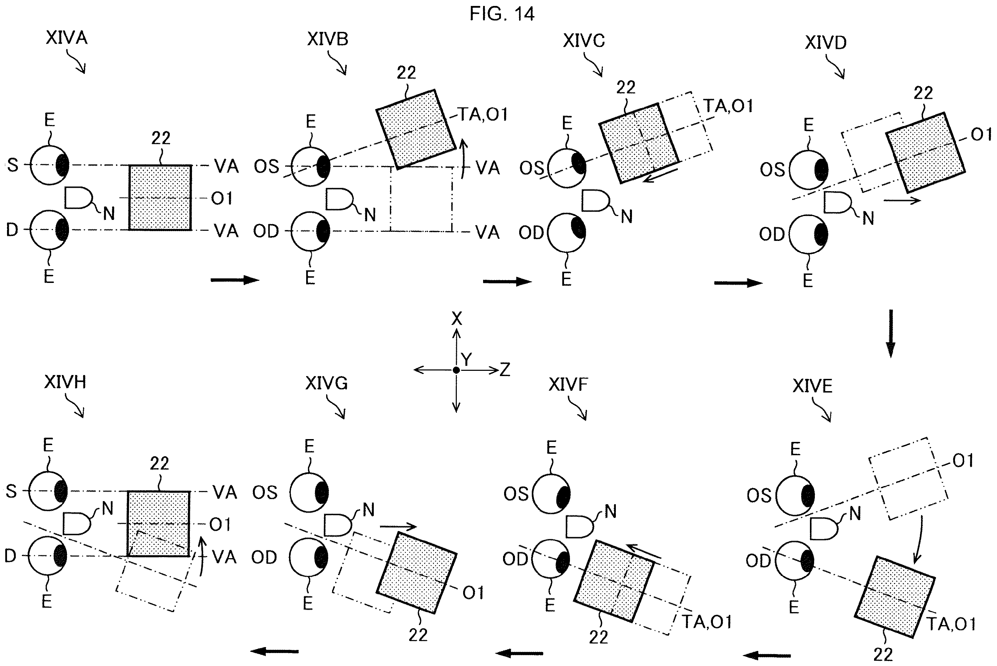

Referring back to Figure 4, when examination on the subject eye E by the examination head 22 (fundus imaging of the subject eye E by the fundus camera unit 24 or OCT imaging of the subject eye E by the OCT unit 26) is completed, the drive controlling unit 46 drives the XZ movement mechanism 16 to retract the examination head 22 toward a rear side in the Z direction (an examiner side) by a predetermined distance (see reference characters XIVD and XIVG in Figure 14 (to be described later)). The drive controlling unit 46 drives at least the swing rotation mechanism 20 (may simultaneously drive the XZ movement mechanism 16 and the Y movement mechanism 18) under control by the retraction controlling unit 47B (to be described later) to retract the examination head 22 from the face (the nose N to be specific) of the subject.

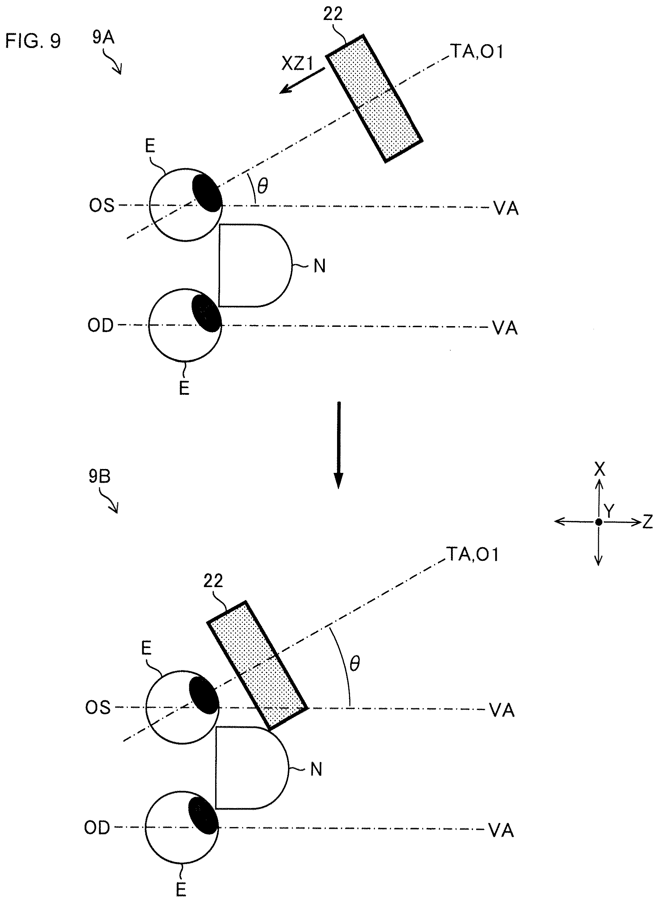

Figure 9 is an explanatory diagram for explaining the likelihood of the examination head 22 coming close to the face (the nose N to be specific) of the subject during the automatic alignment. As indicated by reference character 9A in Figure 9, the automatic alignment displaces the examination head 22 to the examination position for the subject eye E along the axis TA of tilt (see the arrow XZ1 in Figure 9), i.e., brings the examination head 22 closer to the subject eye E. At this time, as indicated by reference character 9B in Figure 9, the examination head 22 may come close to the face, the nose to be specific, of the subject depending on motion of the face or the size and shape of the subject's nose N. For this reason, in the present embodiment, when the examination head 22 is at risk of coming close to the face (the nose N) of the subject while the examination head 22 is displaced to the examination position for the subject eye E along the axis TA of tilt, retraction control that retracts the examination head 22 from the face of the subject is executed.

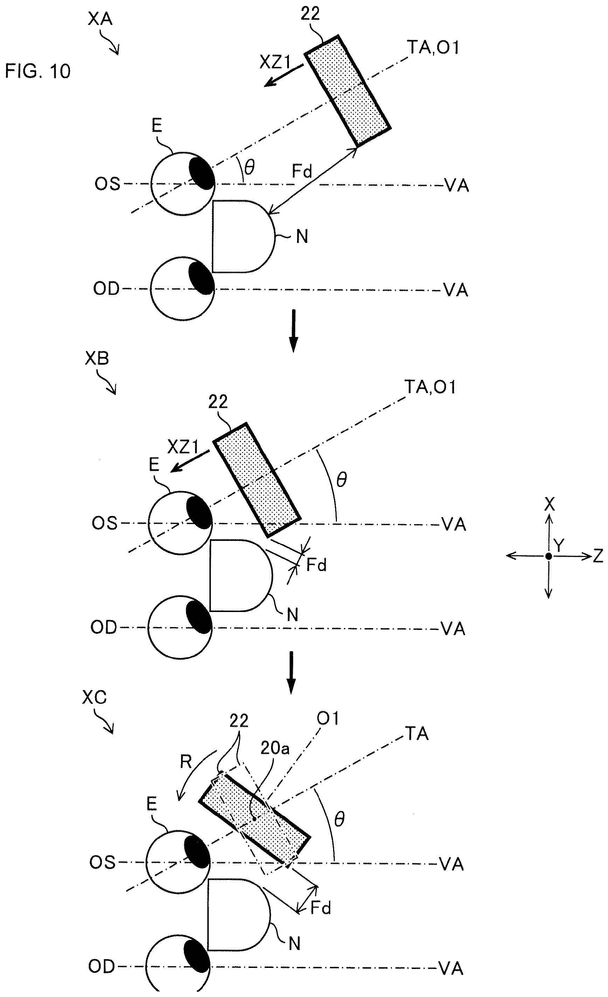

Figure 10 is an explanatory diagram for explaining the retraction control of the examination head 22 according to the first embodiment. As illustrated in Figure 10 and Figure 4, the detection controlling unit 47A, together with the stereo camera 34, constitutes a distance detecting unit according to the presently disclosed subject matter. The detection controlling unit 47A controls the stereo camera 34 to continuously detect a face distance Fd between the examination head 22 and the face of the subject while the examination head 22 is displaced to the examination position for the subject eye E along axis TA of tilt during the automatic alignment. The face distance Fd here is a distance between the examination head 22 and the nose N, to which the examination head 22 is highly likely to come closer, in the face of the subject.

Specifically, the detection controlling unit 47A continuously (repeatedly) execute photographing control that causes the cameras 34a of the stereo camera 34 to photograph the face (the nose N) of the subject from a plurality of directions different from each other and acquisition of photographed face images from the cameras 34a during displacement of the examination head 22 along the axis TA of tilt.

At this time, photographing of the nose N by all the cameras 34a may be impossible depending on photographing conditions (a photographing angle of view and a direction of a photographing optical axis O2) of each camera 34a of the stereo camera 34. For example, when the subject eye E is the left eye OS, the nose N may be photographable only by the camera 34a located on the right of the objective lens 30 as viewed from the left eye OS among the cameras 34a or may be photographable only by the cameras 34a located on the right of and below the objective lens 30.

In such a case, the detection controlling unit 47A continuously executes photographing of the face (the nose N) by one or a plurality of cameras 34a capable of photographing the nose N on the axis TA of tilt and acquisition of photographed images from the one or plurality of cameras 34a that have photographed the nose N. The cameras 34a capable of photographing the nose N on the axis TA of tilt in the stereo camera 34 can be determined based on left and right eye information indicating whether the subject eye E is the left eye OS or the right eye OD. The phrase "the plurality of cameras 34a" here does not always mean all the cameras 34a.

When the face (the nose N) is photographed by a plurality of cameras 34a, each time photographed face images are acquired from the cameras 34a, the detection controlling unit 47A repeatedly executes computation of the face distance Fd based on the photographed face images from the cameras 34a. For example, the detection controlling unit 47A analyzes a photographed image from each camera 34a by a publicly known method and identifies a nose contour from the background, identifies a cheeks contour of the subject from the background, or identifies a nostril, thereby identifying an image of the nose N (a tip) in the photographed image.

The detection controlling unit 47A then computes the face distance Fd (the relative position of the nose N to the examination head 22) that is a shortest distance from the examination head 22 to the nose N based on a result of the identification of the images of the nose N in the respective photographed images from the cameras 34a. A method for computing distances to various objects using the stereo camera 34 is a publicly known technique (see, for example, Patent Literature 1), and a specific description thereof will be omitted. With this configuration, the face distance Fd can be continuously detected while the examination head 22 is displaced to the examination position for the subject eye E along the axis TA of tilt (see reference character XA in Figure 10).

When the number of cameras 34a capable of photographing the nose N on the axis TA of tilt is one, the detection controlling unit 47A executes photographing of the nose N by the camera 34a (corresponding to a particular camera according to the presently disclosed subject matter) at the initial position for the examination head 22 to acquire a photographed image of the nose N. In this case, the detection controlling unit 47A functions as a prior photographing controlling unit according to the presently disclosed subject matter.

Each time the detection controlling unit 47A acquires a new photographed image of the nose N from the one camera 34a that has photographed the nose N during displacement of the examination head 22 along the axis TA of tilt, the detection controlling unit 47A executes a computation process of comparing the size of a contour of an image of the nose N included in the photographed image of the nose N with that of a contour of an image of the nose N included in the photographed image of the nose N at the initial position (obtaining a ratio therebetween) to compute the face distance Fd. This process allows continuous detection of the face distance Fd.

The retraction controlling unit 47B executes the retraction control that drives the swing rotation mechanism 20 to retract the examination head 22 from the face (the nose N) of the subject when the face distance Fd is less than a threshold determined in advance, based on the face distance Fd continuously detected by the detection controlling unit 47A while the examination head 22 is displaced to the examination position for the subject eye E along the axis TA of tilt (see reference character XB in Figure 10). Specifically, the retraction controlling unit 47B drives the swing rotation mechanism 20, as indicated by reference character XC in Figure 10, to execute the retraction control that rotates the examination head 22 in a direction of increasing the face distance Fd (see the arrow R), as indicated by reference character XC in Figure 10. A rotation direction for the examination head 22 which increases the face distance Fd can be determined based on the information such as the above-described left and right eye information and how the outward direction X1 is oriented (see reference character 6A in Figure 6).

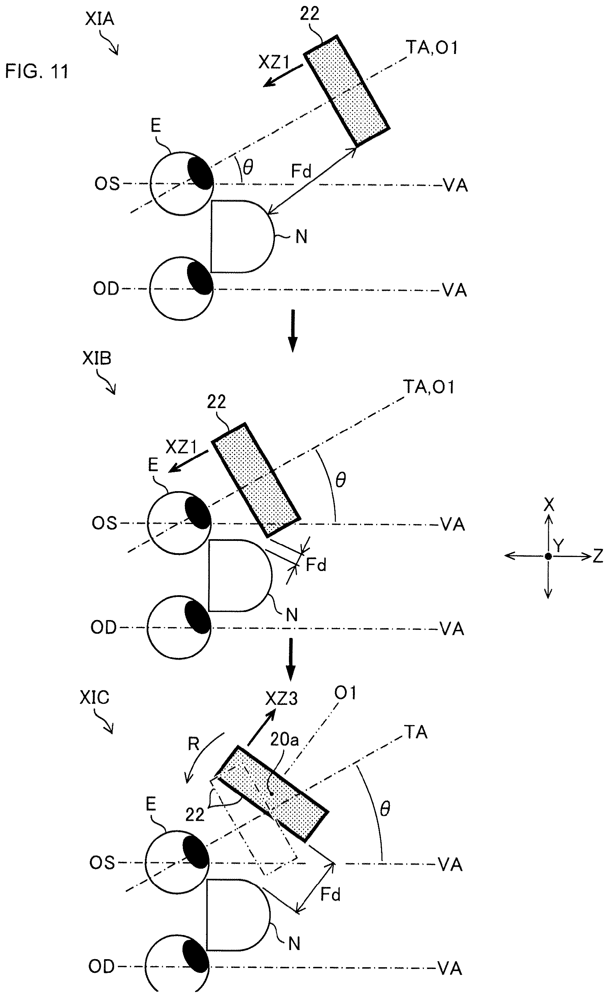

Figure 11 is an explanatory diagram for explaining a modification of the retraction control of the examination head 22 according to the first embodiment. As indicated by reference characters XIA and XIB in Figure 11, the retraction controlling unit 47B may drive both the swing rotation mechanism 20 and the XZ movement mechanism 16 to execute the retraction control when the face distance Fd continuously detected by the detection controlling unit 47A is less than the threshold determined in advance.

Specifically, as indicated by reference character XIC in Figure 11, the retraction controlling unit 47B simultaneously executes first retraction control and second retraction control as the retraction control. In the first retraction control, the retraction controlling unit 47B drives the swing rotation mechanism 20 to rotate the examination head 22 in the direction of increasing the face distance Fd (see the arrow R), as described with reference to Figure 10. In the second retraction control, the retraction controlling unit 47B drives the XZ movement mechanism 16 to move the examination head 22 in a direction (at least one direction of the X direction and the Z direction) away from the face (the nose N) of the subject (see an arrow XZ3). Additionally, the retraction controlling unit 47B may drive the XZ movement mechanism 16 without driving the swing rotation mechanism 20 to retract the examination head 22 in the direction (at least one direction of the X direction and the Z direction) away from the face of the subject. In this case, the examination head 22 may be retracted, for example, along the axis TA of tilt.

The retraction controlling unit 47B may drive the Y movement mechanism 18 instead of or in addition to the XZ movement mechanism 16 to move the examination head 22 in the Y direction such that the examination head 22 moves away from the face (the nose N) of the subject in executing the second retraction control.

Referring back to Figure 4, when examination on the subject eye E by the examination head 22 (fundus imaging of the subject eye E by the fundus camera unit 24 or OCT imaging of the subject eye E by the OCT unit 26) is completed, the drive controlling unit 46 drives the XZ movement mechanism 16 to retract the examination head 22 toward the rear side in the Z direction (the examiner side) by a predetermined distance (see reference characters XIVD and XIVG in Figure 14 (to be described later)).

The vision fixation controlling unit 48 causes the vision fixation light emitting unit 36 to emit vision fixation light at least during a period from before the start of the automatic alignment of the examination head 22 to completion of examination on the subject eye E by the examination head 22. This makes it possible to guide and fix an eye direction of the subject to a direction of the vision fixation light during movement of the examination head 22 from the initial position to the examination position through the axis TA of tilt at the time of the automatic alignment. For this reason, for example, when the examination head 22 is moved from the initial position to the axis TA of tilt, the subject eye E can be made to circumnutate so as to follow the movement (see Figures 6 to 8). As a result, the eye direction of the subject eye E can be kept fixed to the examination head 22.

The measurement controlling unit 50 controls fundus imaging of the subject eye E by the fundus camera unit 24 and photographing of a tomographic image of the subject eye E by the OCT unit 26. For example, the measurement controlling unit 50 drives a focus optical system (not illustrated) (see Patent Literature 1) housed in the examination head 22 after completion of the automatic alignment of the examination head 22 to execute an autofocusing process of focusing the examination head 22 on a part to be observed (e.g., the fundus) of the subject eye E. The measurement controlling unit 50 then executes fundus imaging of the subject eye E by the fundus camera unit 24 or OCT imaging of the subject eye E by the OCT unit 26.

The measurement controlling unit 50 acquires a fundus image of the subject eye E from the fundus camera unit 24 and outputs the fundus image to the saving controlling unit 52 when fundus imaging of the subject eye E by the fundus camera unit 24 is executed. The measurement controlling unit 50 generates a tomographic image of the subject eye E based on a signal such as a detection signal output from the OCT unit 26 by a publicly known method and outputs the tomographic image to the saving controlling unit 52 when OCT imaging of the subject eye E by the OCT unit 26 is executed.

The saving controlling unit 52 causes the display unit 37 to display a fundus image or a tomographic image of the subject eye E input from the measurement controlling unit 50. The saving controlling unit 52 saves the fundus image or the tomographic image of the subject eye E in the storage unit 39 when the examiner inputs an image saving to the manipulation unit 38.

### [Function of Ophthalmic Device According to First Embodiment]

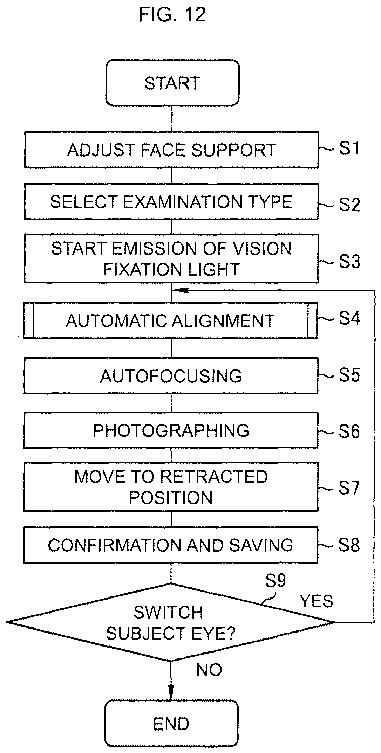

Figure 12 is a flowchart illustrating a flow of a process of examining the subject eye E by the ophthalmic device 10 according to the first embodiment with the above-described configuration. As illustrated in Figure 12, the examiner manipulates the manipulation unit 38 with the subject placing his/her chin against the chin rest 14a and his/her forehead against the forehead rest 14b to adjust height positions (positions in the Y direction) of the chin rest 14a and the forehead rest 14b to fit the subject (step S1).

The examiner then manipulates the manipulation unit 38 to select the type of examination on the subject eye E (fundus imaging by the fundus camera unit 24 or OCT imaging by the OCT unit 26) (step S2). The examiner also manipulates the manipulation unit 38 to select an automatic alignment mode as an alignment mode of the examination head 22. When the examination type of the subject eye E and the alignment mode are selected, the vision fixation controlling unit 48 causes the vision fixation light emitting unit 36 to emit vision fixation light (step S3). This allows guiding and fixation of the eye direction of the subject eye E.

When the examiner inputs an examination start to the manipulation unit 38, the automatic alignment of the examination head 22 with the subject eye E is executed (step S4).

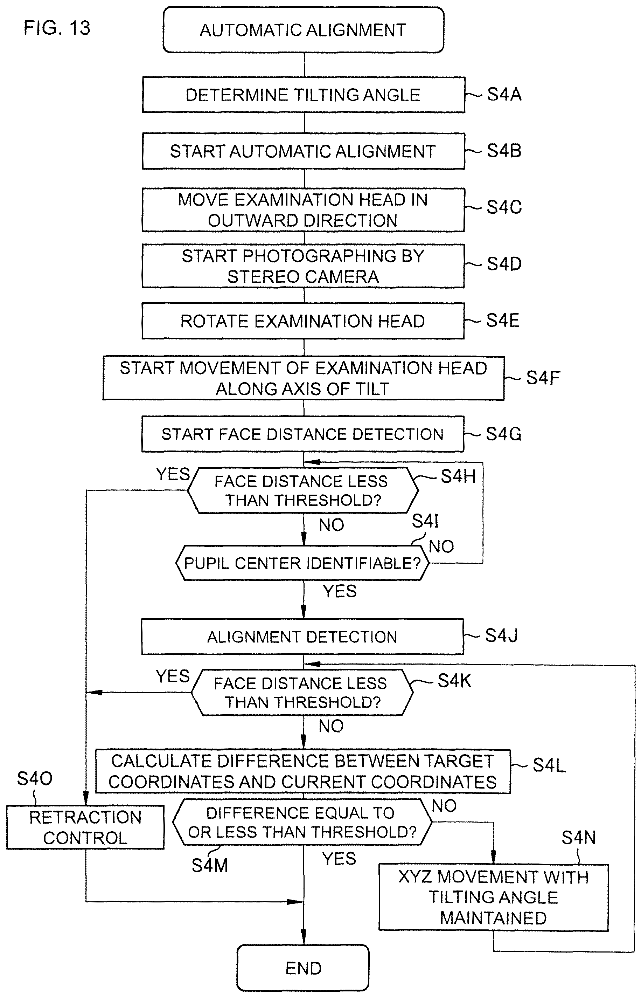

Figure 13 is a flowchart illustrating a flow of an automatic alignment process for the examination head 22 which pertains to a method for operating an ophthalmic device according to the presently disclosed subject matter. Figure 14 is an explanatory diagram for explaining displacement of the examination head 22 after the start of the automatic alignment. The subject eye E is the left eye OS here. And, the example of the automatic alignment in the example 1-1 illustrated in Figure 6 will be described below.

As illustrated in Figures 13 and 14, after the tilting angle determining unit 43 determines, as the tilting angle θ, an angle selected in advance with the manipulation unit 38 by the examiner, the tilting angle determining unit 43 outputs information on the tilting angle θ to the drive controlling unit 46 (step S4A). For this reason, after the drive controlling unit 46 determines the axis TA of tilt based on the tilting angle θ, the drive controlling unit 46 starts the automatic alignment of the examination head 22 (step S4B).

The drive controlling unit 46 drives the XZ movement mechanism 16 to first execute a first driving process of moving the examination head 22 from the initial position to the axis TA of tilt in the outward direction X1 when the examination head 22 is viewed from the one-direction side in the Y direction (step S4C). The alignment detection unit 44 causes the cameras 34a of the stereo camera 34 to start photographing and continuously executes acquisition of photographed images from the cameras 34a and analysis of the photographed images (step S4D).

The drive controlling unit 46 then drives the swing rotation mechanism 20 to execute a second driving process of rotating the examination head 22 around the rotating shaft 20a by the tilting angle θ and making the optical axis O1 of the examination head 22 parallel to the axis TA of tilt (step S4E). With this process, the examination head 22 is displaced from the initial position indicated by reference character XIVA in Figure 14 to the axis TA of tilt, as indicated by reference character XIVB, and the optical axis O1 becomes parallel to the axis TA of tilt. Emission of vision fixation light from the vision fixation light emitting unit 36 allows the eye direction of the subject eye E to follow displacement of the examination head 22.

The drive controlling unit 46 drives the XZ movement mechanism 16 to start a third driving process of moving the examination head 22 to the examination position along the axis TA of tilt when the examination head 22 is viewed from the one-direction side in the Y direction (step S4F, corresponding to a drive controlling step according to the presently disclosed subject matter), as indicated by reference character XIVC in Figure 14. The above-described displacement of the examination head 22 to the examination position along the axis TA of tilt at the time of the automatic alignment prevents the examination head 22 from coming closer to the nose N.

The detection controlling unit 47A continuously executes photographing control that causes one or a plurality of cameras 34a capable of photographing the nose N to photograph the face (the nose N) of the subject and acquisition of photographed face images from the one or plurality of cameras 34a that have photographed the nose N, with the same timing as the start of the third driving process. When the number of cameras 34a capable of photographing the nose N is two or more, the detection controlling unit 47A continuously executes computation of the face distance Fd based on respective photographed face images from the cameras 34a (step S4G, corresponding to a distance detecting step according to the presently disclosed subject matter).

On the other hand, when the nose N is not photographable by a plurality of cameras 34a, each time the detection controlling unit 47A acquires a new photographed image of the nose N from one camera 34a capable of photographing the nose N, the detection controlling unit 47A computes the face distance Fd based on a result of comparing the new photographed image of the nose N with a photographed image of the nose N at the initial position.

The retraction controlling unit 47B remains on standby until the face distance Fd is less than the threshold (NO in step S4H).

While the first driving process to the third driving process are executed, the alignment detection unit 44 waits for a chance for alignment detection (NO in step S4I) until the pupil center position of the subject eye E can be identified from photographed images acquired from the cameras 34a. Halfway through the automatic alignment, the cameras 34a photograph the anterior eye part of the subject eye E, and anterior eye part images of the subject eye E are input as photographed images from the cameras 34a to the alignment detection unit 44. This allows the alignment detection unit 44 to identify the pupil center position of the subject eye E based on the anterior eye part images input from the cameras 34a (YES in step S4I).

The alignment detection unit 44 then executes alignment detection that detects the relative position of the subject eye E to the examination head 22 by converting the pupil center position of the subject eye E into three-dimensional coordinates (step S4J). The alignment detection unit 44 outputs a detection result of the alignment detection to the drive controlling unit 46.

The drive controlling unit 46 switches the alignment mode of the examination head 22 to a manual alignment mode when the alignment detection unit 44 is incapable of alignment detection during a period from the start of the automatic alignment or the start of the third driving process to movement of the examination head 22 for a fixed time determined in advance or over a fixed distance (the same applies to a second embodiment (to be described later)). This prevents the examination head 22 from coming closer to the subject eye E in a state where alignment detection is impossible.

The retraction controlling unit 47B remains on standby even after alignment detection by the alignment detection unit 44 until the face distance Fd detected by the detection controlling unit 47A is less than the threshold (NO in step S4K).

The drive controlling unit 46 drives the XZ movement mechanism 16 and the Y movement mechanism 18 based on the alignment detection result input from the alignment detection unit 44 to continue the third driving process until the examination head 22 reaches the examination position. Specifically, the drive controlling unit 46 calculates a difference between three-dimensional coordinates (target coordinates) of the examination position determined based on the alignment detection result and three-dimensional coordinates of the current examination head 22 (current coordinates) and continues the third driving process until the difference is equal to or less than a threshold (step S4L, NO in step S4M, and step S4N). In this manner, the examination head 22 is moved to the examination position while maintaining the tilting angle θ.

When the difference between the target coordinates and the current coordinates is equal to or less than the threshold while the face distance Fd detected by the detection controlling unit 47A remains equal to or more than the threshold, the drive controlling unit 46 stops driving the XZ movement mechanism 16 and the Y movement mechanism 18 to end the automatic alignment (YES in step S4M).

When the face distance Fd detected by the detection controlling unit 47A is less than the threshold during the third driving process, the retraction controlling unit 47B operates (YES in step S4H or step S4K). As the retraction control, the retraction controlling unit 47B drives the swing rotation mechanism 20 to rotate the examination head 22, as illustrated in Figure 10, or drives the swing rotation mechanism 20 and the XZ movement mechanism 16 to simultaneously execute the first retraction control and the second retraction control, as illustrated in Figure 11 (step S4O). Step S4O corresponds to a retraction controlling step according to the presently disclosed subject matter. This prevents the examination head 22 from coming closer to the nose N due to, e.g., motion of the face of the subject or the shape and size of the subject's nose N.

When the retraction controlling unit 47B executes the retraction control, the tilting angle determining unit 43 may determine a new tilting angle θ (e.g., an angle larger than the tilting angle θ described earlier) and execute again the third driving process along the axis TA of tilt corresponding to the new tilting angle θ, i.e., repeatedly execute the processes in step S4F and the subsequent steps (the same applies to second and subsequent embodiments (to be described later)).

Referring back to Figures 12 and 14, when the automatic alignment is completed, the measurement controlling unit 50 drives the focus optical system (not illustrated) to execute autofocusing (step S5). After that, the measurement controlling unit 50 executes fundus imaging of the subject eye E by the fundus camera unit 24 or OCT imaging of the subject eye E by the OCT unit 26 (step S6). The measurement controlling unit 50 outputs a fundus image of the subject eye E acquired from the fundus camera unit 24 to the saving controlling unit 52 when fundus imaging is executed. The measurement controlling unit 50 generates a tomographic image of the subject eye E based on a signal such as a detection signal output from the OCT unit 26 and outputs the tomographic image to the saving controlling unit 52 when OCT imaging is executed.

When fundus imaging or OCT imaging of the subject eye E is completed, the drive controlling unit 46 drives the XZ movement mechanism 16 to retract the examination head 22 toward the rear side in the Z direction (step S7), as indicated by reference character XIVD in Figure 14.

The saving controlling unit 52 causes the display unit 37 to display the fundus image or the tomographic image of the subject eye E input from the measurement controlling unit 50. This allows the examiner to confirm whether a desired fundus image or tomographic image is obtained. When a desired fundus image or tomographic image is obtained, the examiner inputs an image saving to the manipulation unit 38. With this manipulation, the saving controlling unit 52 saves the fundus image or tomographic image of the subject eye E in the storage unit 39 (step S8).

When the examiner proceeds to examine the right eye OD, the processes in step S4 to step S8 are repeatedly executed (YES in step S9). In this case, the examination head 22 is displaced to the axis TA of tilt corresponding to the right eye OD (see reference character XIVE in Figure 14) and is further moved to an examination position for the right eye OD along the axis TA of tilt (see reference character XIVF in Figure 14), under control by the drive controlling unit 46. When the examination on the right eye OD is completed, the examination head 22 is retracted toward the rear side in the Z direction (see reference character XIVG in Figure 14) and is then displaced to the initial position (see reference character XIVH in Figure 14), under the control by the drive controlling unit 46.

As described above, in the ophthalmic device 10 according to the first embodiment, when the face distance Fd becomes less than the threshold halfway through movement of the examination head 22 to the examination position for the subject eye E along the axis TA of tilt at the time of the automatic alignment, the retraction control of the examination head 22 can be executed by driving at least the swing rotation mechanism 20. This makes it possible to reliably avoid the examination head 22 coming closer to the nose N during the automatic alignment, regardless of motion of the face of the subject or the shape and size of the subject's nose N.

### [Second Embodiment]

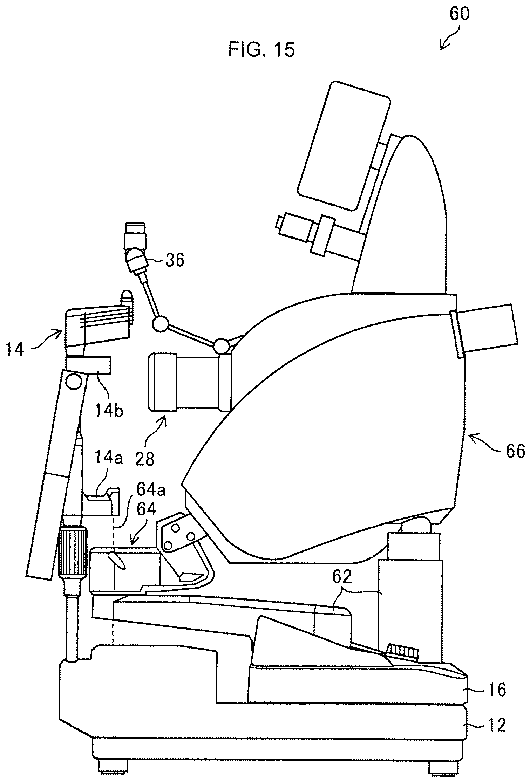

Figure 15 is a side view of an ophthalmic device 60 according to a second embodiment. While the examination head 22 is rotated (swung) around the objective lens 30 by the swing rotation mechanism 20 (the rotating shaft 20a) in the ophthalmic device 10 according to the above-described first embodiment, the ophthalmic device 60 according to the second embodiment includes an examination head 66 different in rotation center position from that in the first embodiment.

Components functionally or structurally identical to those in the ophthalmic device 10 according to the first embodiment are denoted by identical reference numerals, and a description thereof will be omitted.

As illustrated in Figure 15, the ophthalmic device 60 is a fundus camera and includes a base 12, a face support 14, an XZ movement mechanism 16, a vision fixation light emitting unit 36 (only an external fixation lamp of which is illustrated), a Y movement mechanism 62, a swing rotation mechanism 64, and the examination head 66. The ophthalmic device 60 also includes a stereo camera 34, a display unit 37, a manipulation unit 38, a storage unit 39, and a control device 40 (all not illustrated) described in the first embodiment.

The Y movement mechanism 62, together with the XZ movement mechanism 16, constitutes a movement mechanism according to the presently disclosed subject matter. The Y movement mechanism 62 has a shape extending toward a front side in a Z direction. The swing rotation mechanism 64 is provided at a distal end portion on the front side in the Z direction of the Y movement mechanism 62. The Y movement mechanism 62 integrally moves the swing rotation mechanism 64 and the examination head 66 in a Y direction. The XZ movement mechanism 16 and the Y movement mechanism 62 are capable of integrally moving the swing rotation mechanism 64 and the examination head 66 in an X direction and the Y and Z directions.

The swing rotation mechanism 64 corresponds to a rotation mechanism according to the presently disclosed subject matter and, together with the XZ movement mechanism 16 and the Y movement mechanism 62, constitutes a displacement mechanism according to the presently disclosed subject matter. The swing rotation mechanism 64 has a rotation axis 64a parallel to the Y direction and rotates the examination head 66 around the rotation axis 64a. The rotation axis 64a is provided in front of a lens-barrel 28 (an objective lens 30) of the examination head 66 in the Z direction. With this configuration, the rotation axis 64a and a subject eye E (a circumnutation center) can be made to coincide when viewed from a one-direction side in the Y direction by adjusting X and Z positions of the swing rotation mechanism 64 by the XZ movement mechanism 16. In this case, the swing rotation mechanism 64 rotates (swings) the examination head 66 around the circumnutation center of the subject eye E.

The swing rotation mechanism 64 can also rotate (tilt) the examination head 66 around a rotation axis perpendicular to the Y direction. The rotation is not illustrated.

The examination head 66 is attached to the swing rotation mechanism 64. With this configuration, the examination head 66 is movable in the X, Y, and Z directions by the XZ movement mechanism 16 and the Y movement mechanism 62 and is rotatable in a direction around the rotation axis 64a by the swing rotation mechanism 64. The examination head 66 includes a fundus camera unit 24 and the lens-barrel 28 (including the stereo camera 34) described in the first embodiment.

The control device 40 according to the second embodiment is basically the same as the control device 40 according to the first embodiment except that a method for automatic alignment of the examination head 66 by a drive controlling unit 46 is different.

The drive controlling unit 46 according to the second embodiment determines an axis TA of tilt based on a tilting angle 0 determined by a tilting angle determining unit 43 and then drives the XZ movement mechanism 16, the Y movement mechanism 62, and the swing rotation mechanism 64 to execute the automatic alignment of the examination head 66, like the first embodiment.

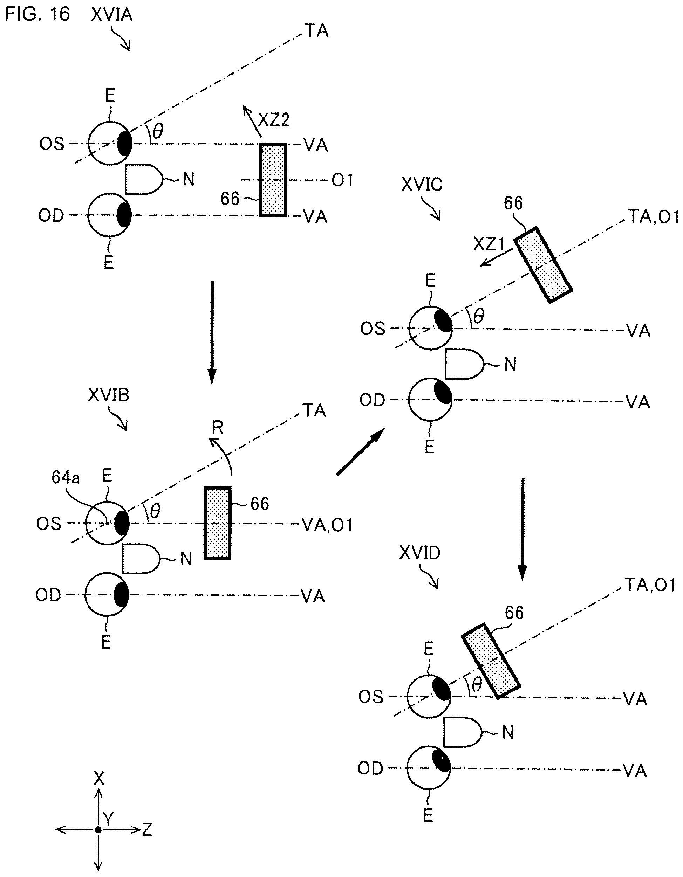

Figure 16 is an explanatory diagram for explaining an example 2-1 of the automatic alignment of the examination head 66 according to the second embodiment. As indicated by reference character XVIA in Figure 16, the examination head 66 is arranged at the same initial position as the first embodiment at first.

As indicated by reference characters XVIA and XVIB in Figure 16, the drive controlling unit 46 drives the XZ movement mechanism 16 to execute a first driving process of moving the examination head 66 (the swing rotation mechanism 64) from the initial position in the X and Z directions (see an arrow XZ2). Specifically, the examination head 66 is moved in the X and Z directions to a position where the rotation axis 64a coincides with the circumnutation center of the subject eye E when viewed from the one-direction side in the Y direction.

The drive controlling unit 46 drives the swing rotation mechanism 20 after completion of the first driving process to execute a second driving process of rotating the examination head 66 around the rotating shaft 20a (the circumnutation center of the subject eye E) by the tilting angle θ (see an arrow R). With this process, as indicated by reference character XVIC in Figure 16, the examination head 66 is moved to the axis TA of tilt, and an optical axis O1 becomes parallel to the axis TA of tilt.

The drive controlling unit 46 then drives the XZ movement mechanism 16 after completion of the second driving process to start a third driving process of moving the examination head 66 to an examination position along the axis TA of tilt when the examination head 66 is viewed from the one-direction side in the Y direction (see an arrow XZ1), like the first embodiment. In the third driving process according to the second embodiment, Z-axis movement of the examination head 66 by the XZ movement mechanism 16 is mainly executed, unlike the first embodiment. With this movement, the examination head 66 is moved toward the subject eye E while keeping the tilting angle θ constant.

As indicated by reference character XVID in Figure 16, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position, unless retraction control is executed.

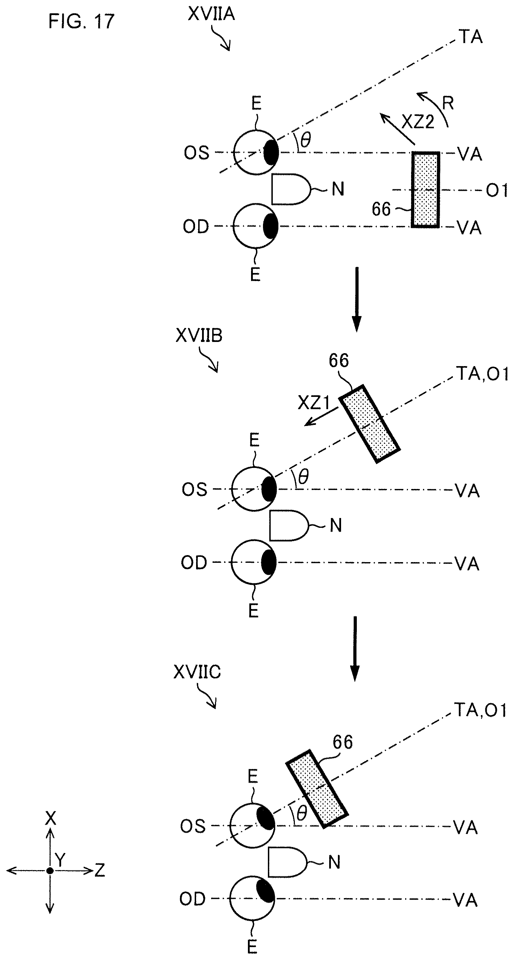

Figure 17 is an explanatory diagram for explaining an example 2-2 of the automatic alignment of the examination head 66 according to the second embodiment. As indicated by reference characters XVIIA and XVIIB in Figure 17, the drive controlling unit 46 simultaneously drives the XZ movement mechanism 16, the Y movement mechanism 62, and the swing rotation mechanism 64 to execute a first driving process of simultaneously executing movement of the examination head 66 in the X and Z directions and rotation of the examination head 66 by the tilting angle θ (see the arrow XZ2 and the arrow R). The first driving process in the example 2-2 is a process of simultaneously executing the first driving process and the second driving process in the example 2-1 described with reference to Figure 16. With this process, the examination head 66 is moved to the axis TA of tilt, and the optical axis O1 of the objective lens 30 becomes parallel to the axis TA of tilt.

In the first driving process, the examination head 66 may be displaced to a position on the axis TA of tilt where the stereo camera 34 can photograph an anterior eye part of the subject eye E or a position where an observation optical system (not illustrated) inside the examination head 22 can photograph the anterior eye part of the subject eye E via a shortest route.

The drive controlling unit 46 drives the XZ movement mechanism 16 and the Y movement mechanism 62 after completion of the first driving process to execute a second driving process which is the same as the third driving process in the example 2-1, thereby moving the examination head 66 to the examination position along the axis TA of tilt when the examination head 66 is viewed from the one-direction side in the Y direction (see the arrow XZ1). As indicated by reference character XVIIC in Figure 17, the drive controlling unit 46 drives the XZ movement mechanism 16 and the Y movement mechanism 62 based on alignment detection performed by the alignment detection unit 44 halfway through the automatic alignment to continue the second driving process until the examination head 66 reaches the examination position, unless the retraction control is executed.

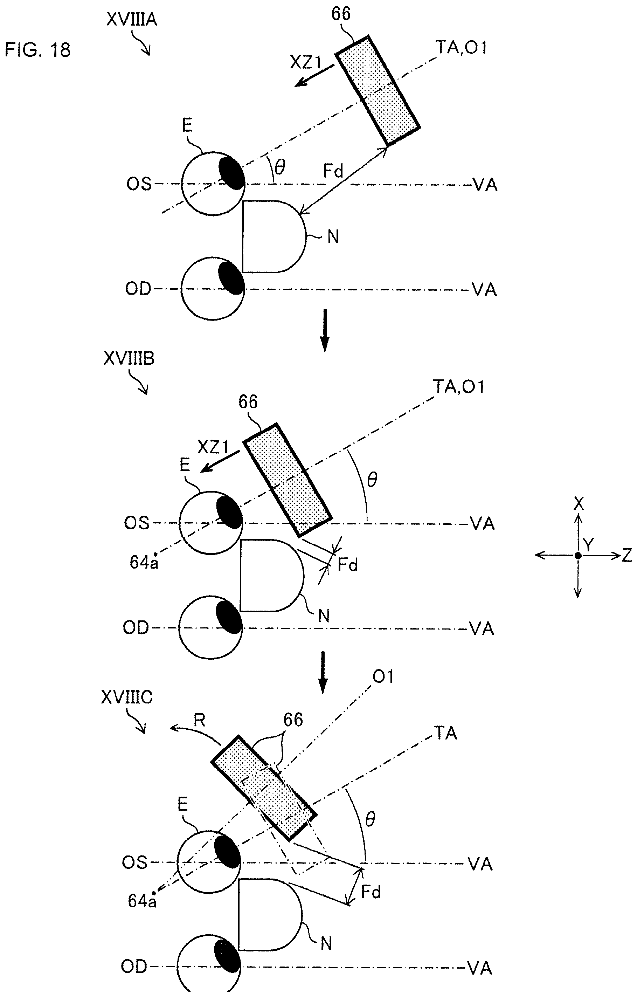

Figure 18 is an explanatory diagram for explaining the retraction control of the examination head 66 according to the second embodiment. As indicated by reference characters XVIIIA and XVIIIB in Figure 18, a detection controlling unit 47A according to the second embodiment continuously executes computation of a face distance Fd while the examination head 66 is displaced to the examination position for the subject eye E along the axis TA of tilt, like the first embodiment.

A retraction controlling unit 47B according to the second embodiment executes the retraction control that drives the swing rotation mechanism 64 to rotate the examination head 66 in a direction of increasing the face distance Fd (see the arrow R) around the rotation axis 64a when the face distance Fd detected by the detection controlling unit 47A is less than a threshold determined in advance. The retraction controlling unit 47B may drive at least one of the XZ movement mechanism 16 and the Y movement mechanism 62 together with the swing rotation mechanism 64 to move the examination head 66 in the direction of increasing the face distance Fd while rotating the examination head 66, as described with reference to Figure 11.

As described above, in the ophthalmic device 10 according to the second embodiment, it is possible to reliably avoid the examination head 22 coming closer to a nose N during the automatic alignment by executing the retraction control when the face distance Fd is less than the threshold determined in advance, like the above-described first embodiment.

### [Third Embodiment]

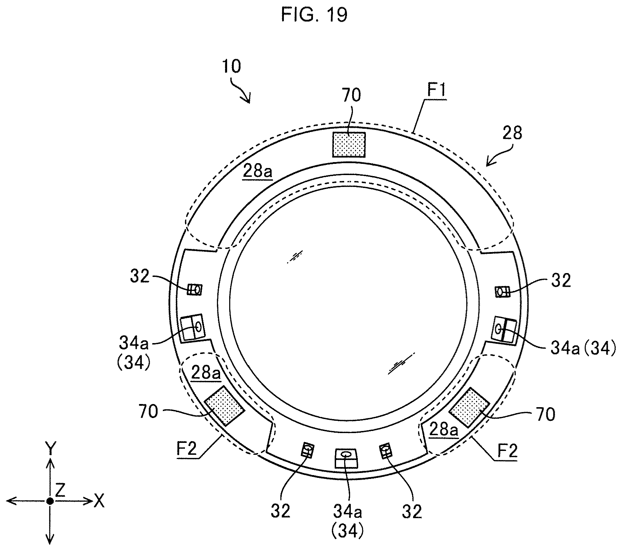

Figure 19 is a front view of a lens-barrel distal end face 28a of an ophthalmic device 10 according to a third embodiment. Although the detection controlling unit 47A in the ophthalmic device 10 according to each of the above-described embodiments uses the stereo camera 34 to detect the face distance Fd, the face distance Fd may be detected using another method.

As illustrated in Figure 19, the ophthalmic device 10 according to the third embodiment is basically the same in configuration as the ophthalmic devices 10 according to the embodiments (the same applies to fourth and fifth embodiments (to be described later)) except that the ophthalmic device 10 includes a plurality of non-contact distance sensors 70. For this reason, components functionally or structurally identical to those in the ophthalmic devices 10 according to the embodiments are denoted by identical reference numerals, and a description thereof will be omitted.

The distance sensors 70, together with the detection controlling unit 74A, constitute a distance detecting unit according to the presently disclosed subject matter and are provided, for example, at the lens-barrel distal end face 28a. Each distance sensor 70 may be provided at a site other than the lens-barrel distal end face 28a of a lens-barrel 28 or may be provided at a front surface of a housing of an examination head 22 or 66. The number of distance sensors 70 may be one. Various publicly known distance sensors, such as a photoelectric sensor, an optical fiber sensor, a laser sensor, a camera-equipped laser displacement sensor, an ultrasonic sensor, or a capacitance type sensor, are used as the distance sensors 70.

A detection controlling unit 47A according to the third embodiment continuously executes acquisition of detection signals output from the distance sensors 70 and computation of the face distance Fd based on the detection signals from the distance sensors 70 while the examination head 22 is displaced to an examination position for a subject eye E along an axis TA of tilt. This allows a retraction controlling unit 47B according to the third embodiment to drive at least a swing rotation mechanism 64 to execute the same retraction control of the examination head 22 or 66 as the embodiments when the face distance Fd detected by the detection controlling unit 47A is less than a threshold determined in advance. As a result, the same effects as those of the embodiments can be obtained. The third embodiment is also effective when the face distance Fd is less than the threshold due to a shift in a position of a face of a subject during automatic alignment.

### [Fourth Embodiment]

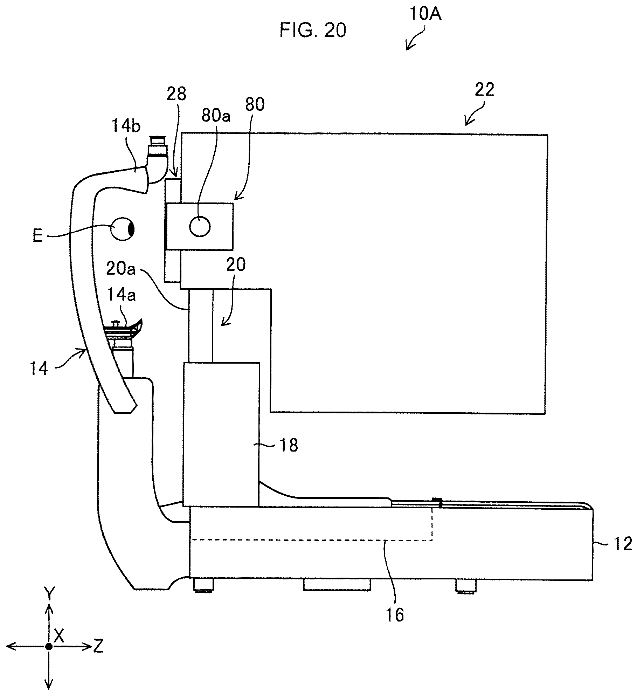

Figure 20 is a side view of an ophthalmic device 10A according to a fourth embodiment. The ophthalmic device 10 (see Figure 1) according to the above-described first embodiment includes the swing rotation mechanism 20 having the rotating shaft 20a parallel to the Y direction, and brings the examination head 22 closer to the subject eye E along the axis TA of tilt obtained by tilting the reference axis VA in the X direction (the outward direction X1) around the subject eye E at the time of automatic alignment of the examination head 22. In contrast, the ophthalmic device 10A according to the fourth embodiment brings an examination head 22 closer to a subject eye E along an axis TA of tilt obtained by tilting a reference axis VA in a direction other than an X direction around a subject eye E at the time of automatic alignment of the examination head 22.

As illustrated in Figure 20, the ophthalmic device 10A according to the fourth embodiment is basically the same in configuration as the ophthalmic device 10 according to the first embodiment except that the ophthalmic device 10A includes a tilt rotation mechanism 80 and executes the automatic alignment of the examination head 22 differently from the first embodiment. For this reason, components functionally or structurally identical to those in the ophthalmic device 10 according to the first embodiment are denoted by identical reference numerals, and a description thereof will be omitted.

The tilt rotation mechanism 80 corresponds to a rotation mechanism according to the presently disclosed subject matter and, together with an XZ movement mechanism 16, a Y movement mechanism 18, and a swing rotation mechanism 20, constitutes a displacement mechanism according to the presently disclosed subject matter. The tilt rotation mechanism 80 includes a rotating shaft 80a parallel to a Y direction and an electric drive mechanism which rotates the rotating shaft 80a, and rotates (tilts) the examination head 22 around the rotating shaft 80a.

A position of the rotating shaft 80a and a position of an objective lens 30 coincide (the term "coincide" as used herein is intended to include the meaning of "coincide substantially"; the same applies hereinafter) when the rotating shaft 80a is viewed from a one-direction side in an axial direction of the rotating shaft 80a. With this configuration, the examination head 22 is rotated (tilted) around the objective lens 30 by the tilt rotation mechanism 80.

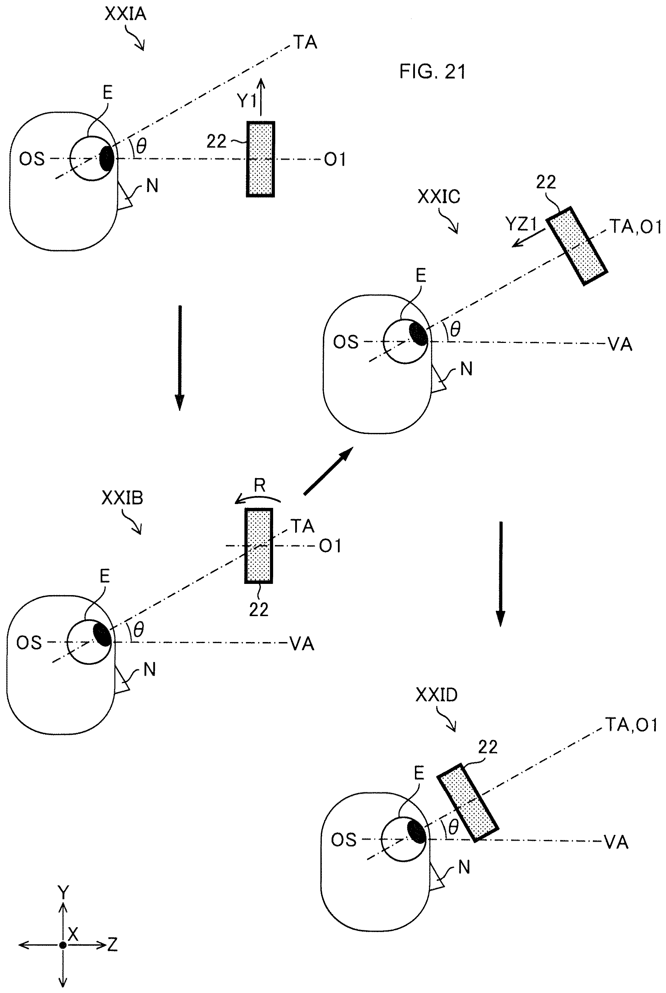

As described above, the examination head 22 according to the fourth embodiment is biaxially rotatable (swingable and tiltable) around the objective lens 30 by the swing rotation mechanism 20 and the tilt rotation mechanism 80. The examination head 22 according to the fourth embodiment is thus rotatable around an arbitrary rotating shaft (including ones other than a rotating shaft 20a and the rotating shaft 80a) perpendicular to a Z direction by driving at least one of the swing rotation mechanism 20 and the tilt rotation mechanism 80. For this reason, in the fourth embodiment, a direction (which is a direction perpendicular to the Z direction and away from a nose N) other than the outward direction X1 according to the first embodiment, such as an upward direction of the Y direction, is set as an outward direction Y1 (see Figure 21), and an axis obtained by tilting the reference axis VA in the outward direction Y1 around the subject eye E is set as the axis TA of tilt.

A control device 40 according to the fourth embodiment is basically the same as the control device 40 according to the first embodiment except that a direction of tilt of the axis TA of tilt is different from that in the first embodiment.

A tilting angle determining unit 43 according to the fourth embodiment determines a tilting angle θ in the outward direction Y1 (see Figure 21) of the axis TA of tilt with respect to the reference axis VA when viewed from a one-direction side in the X direction, i.e., the tilting angle θ of the axis TA of tilt with respect to the reference axis VA in a YZ plane. A specific method for determining the tilting angle θ is the same as the method for determining the tilting angle θ according to the first embodiment except that the direction of tilt of the axis TA of tilt is different and that a specific description thereof will be omitted.

A drive controlling unit 46 according to the fourth embodiment determines the axis TA of tilt based on the tilting angle θ determined by the tilting angle determining unit 43 and then drives the XZ movement mechanism 16, the Y movement mechanism 18, the swing rotation mechanism 20, and the tilt rotation mechanism 80 to execute the automatic alignment of the examination head 22.

Figure 21 is an explanatory diagram for explaining an example 3 of the automatic alignment of the examination head 22 according to the fourth embodiment. The example 3 is basically the same as the example 1-1 (see Figure 6) described in the first embodiment except that the direction of tilt of the axis TA of tilt is different.

As indicated by reference character XXIA in Figure 21, the examination head 22 is arranged at the same initial position as the first embodiment at first. The drive controlling unit 46 according to the fourth embodiment drives the XZ movement mechanism 16 and the Y movement mechanism 18 to execute a first driving process of moving the examination head 22 from the initial position to the axis TA of tilt in the outward direction Y1 (the upward direction of the Y direction) when the examination head 22 is viewed from the one-direction side in the X direction.

As indicated by reference character XXIB in Figure 21, the drive controlling unit 46 drives the tilt rotation mechanism 80 after completion of the first driving process to execute a second driving process of rotating the examination head 22 around the rotating shaft 80a by the tilting angle θ (see an arrow R). With this process, as indicated by reference character XXIC in Figure 21, an optical axis O1 of the examination head 22 (the objective lens 30) becomes parallel to the axis TA of tilt. The second driving process may be executed before the first driving process.

The drive controlling unit 46 then drives the XZ movement mechanism 16 and the Y movement mechanism 18 after completion of the second driving process to start a third driving process of moving the examination head 22 to an examination position along the axis TA of tilt when the examination head 22 is viewed from the one-direction side in the X direction (see an arrow YZ1). With this process, the examination head 22 is moved toward the subject eye E while keeping the tilting angle θ constant (the term "constant" as used herein is intended to include the meaning of "substantially constant"; the same applies hereinafter).

As indicated by reference character XXID in Figure 21, the drive controlling unit 46 drives the XZ movement mechanism 16 and the Y movement mechanism 18 based on alignment detection performed by an alignment detection unit 44 halfway through the automatic alignment to continue the third driving process until the examination head 22 reaches the examination position.

The drive controlling unit 46 switches an alignment mode of the examination head 22 to a manual alignment mode when the alignment detection unit 44 is incapable of alignment detection during a period from the start of the automatic alignment or the start of the third driving process to movement of the examination head 22 for a fixed time determined in advance or over a fixed distance.

Like the example 1-2 (see Figure 7) of the automatic alignment of the examination head 22 according to the first embodiment, the first driving process may be started after the examination head 22 is first moved toward a front side in the Z direction (a subject eye E side) by a predetermined distance. Like the example 1-3 (see Figure 8) of the automatic alignment of the examination head 22 according to the first embodiment, a first driving process of simultaneously executing movement of the examination head 22 toward the front side in the Z direction and in the outward direction Y1 and rotation of the examination head 22 by the tilting angle θ may be executed.

As described above, in the ophthalmic device 10 according to the fourth embodiment as well, the examination head 22 can be moved to the examination position for the subject eye E from an oblique direction (obliquely upward direction) along the axis TA of tilt at the time of the automatic alignment. As a result, the same effects as those of the first embodiment can be achieved.

Although the ophthalmic device 10A brings the examination head 22 closer to the subject eye E along the axis TA of tilt obtained by tilting the reference axis VA in the outward direction Y1 (the upward direction of the Y direction) around the subject eye E at the time of the automatic alignment of the examination head 22 in the fourth embodiment, the direction of tilt of the axis TA of tilt is not particularly limited as long as the direction is a direction perpendicular to the Z direction and away from the nose N. Directions of the rotating shafts 20a and 80a may be appropriately changed in accordance with the direction of tilt.

When the direction of tilt of the axis TA of tilt is fixed to the outward direction Y1 in the ophthalmic device 10A according to the fourth embodiment, the swing rotation mechanism 20 may be omitted.

### [Fifth Embodiment]

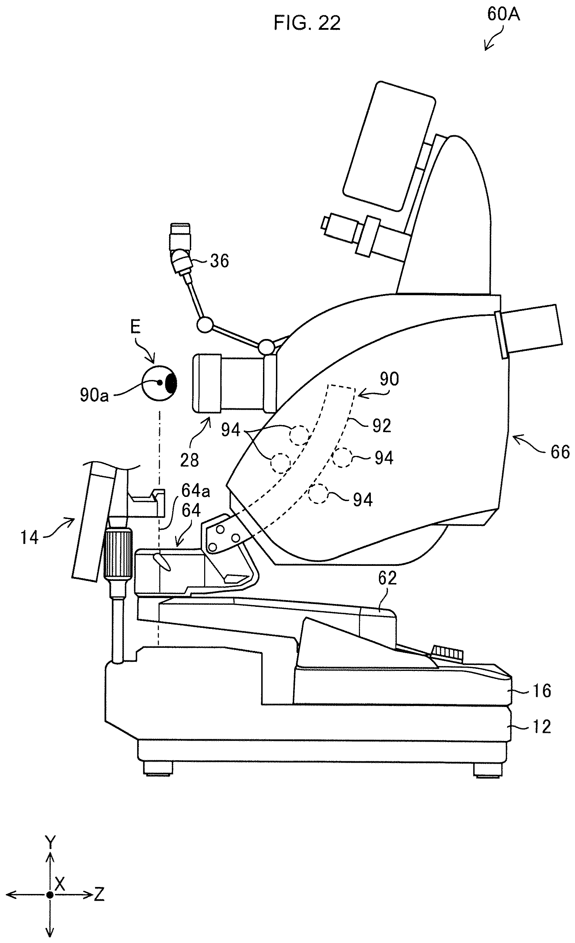

Figure 22 is a side view of an ophthalmic device 60A according to a fifth embodiment. The ophthalmic device 10 (see Figure 15) according to the above-described second embodiment includes the swing rotation mechanism 64 having the rotation axis 64a (corresponding to a first rotation axis according to the presently disclosed subject matter) parallel to the Y direction, and brings the examination head 66 closer to the subject eye E along the axis TA of tilt obtained by tilting the reference axis VA in the X direction (the outward direction X1) around the subject eye E at the time of the automatic alignment of the examination head 66. In contrast, the ophthalmic device 60A according to the fifth embodiment brings an examination head 66 closer to a subject eye E along an axis TA of tilt obtained by tilting a reference axis VA in a direction other than an X direction around the subject eye E at the time of automatic alignment of the examination head 66, like the ophthalmic device 10A according to the above-described fourth embodiment.

As illustrated in Figure 22, the ophthalmic device 60A according to the fifth embodiment is basically the same in configuration as the ophthalmic device 60 according to the second embodiment except that the ophthalmic device 60A includes a tilt rotation mechanism 90 and executes the automatic alignment of the examination head 66 differently from the second embodiment. For this reason, components functionally or structurally identical to those in the ophthalmic device 60 according to the second embodiment are denoted by identical reference numerals, and a description thereof will be omitted.

The tilt rotation mechanism 90 corresponds to a rotation mechanism according to the presently disclosed subject matter and, together with an XZ movement mechanism 16, a Y movement mechanism 62, and a swing rotation mechanism 64, constitutes a displacement mechanism according to the presently disclosed subject matter. The tilt rotation mechanism 90 rotates (tilts) the examination head 66 around an imaginary rotation axis 90a (corresponding to a second rotation axis according to the presently disclosed subject matter) perpendicular to a Y direction.

The tilt rotation mechanism 90 includes, for example, a curved arm 92, a plurality of guide wheel portions 93, and a head moving mechanism (not illustrated). The curved arm 92 is fixed to the swing rotation mechanism 64 and has the shape of an arc of a circle centered at the rotation axis 90a.

The plurality of guide wheel portions 93 are held inside the examination head 66 so as to be rotatable around center axes parallel to the rotation axis 90a. The guide wheel portions 93 are arranged such that the curved arm 92 is sandwiched therebetween in the Y direction. With this configuration, the examination head 66 is movable along the curved arm 92.

The head moving mechanism (not illustrated) of the tilt rotation mechanism 90 is provided inside the examination head 66 and moves the examination head 66 along the curved arm 92. A configuration of the head moving mechanism is a publicly known technique (e.g., Japanese Patent Application Laid-Open No. 2022-112637) and that a specific description thereof will be omitted. The movement of the examination head 66 along the curved arm 92 by the head moving mechanism allows rotation (tilting) of the examination head 66 around the rotation axis 90a. Additionally, alignment of the rotation axis 90a with the subject eye E allows rotation (tilting) of the examination head 66 around the subject eye E.

As described above, the examination head 66 according to the fifth embodiment is biaxially rotatable (swingable and tiltable) by the swing rotation mechanism 64 and the tilt rotation mechanism 90, like the examination head 22 according to the fourth embodiment. The examination head 66 according to the fifth embodiment is thus rotatable around an arbitrary rotation axis (including ones other than a rotation axis 64a and the rotation axis 90a) perpendicular to a Z direction by driving at least one of the swing rotation mechanism 64 and the tilt rotation mechanism 90. For this reason, in the fifth embodiment, a direction (which is a direction perpendicular to the Z direction and away from a nose N) other than the outward direction X1 according to the second embodiment, such as an upward direction of the Y direction, is set as an outward direction Y1, and an axis obtained by tilting the reference axis VA in the outward direction Y1 around the subject eye E is set as the axis TA of tilt, like the fourth embodiment.

A control device 40 according to the fifth embodiment is basically the same as the control device 40 according to the second embodiment except that a direction of tilt of the axis TA of tilt is different from that in the second embodiment.

A tilting angle determining unit 43 according to the fifth embodiment determines a tilting angle θ in the outward direction Y1 of the axis TA of tilt with respect to the reference axis VA, like the tilting angle determining unit 43 according to the fourth embodiment.

A drive controlling unit 46 according to the fifth embodiment determines the axis TA of tilt based on the tilting angle θ determined by the tilting angle determining unit 43 and then drives the XZ movement mechanism 16, the Y movement mechanism 62, the swing rotation mechanism 64, and the tilt rotation mechanism 90 to execute the automatic alignment of the examination head 66.

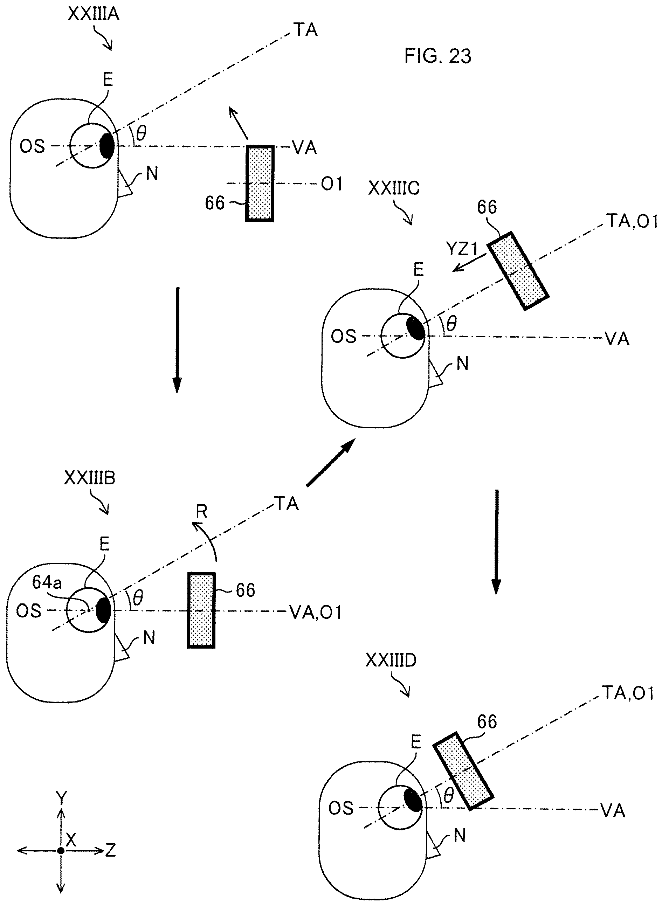

Figure 23 is an explanatory diagram for explaining an example 5 of the automatic alignment of the examination head 66 according to the fifth embodiment. The example 5 is basically the same as the example 2-1 (see Figure 16) described in the second embodiment except that the direction of tilt of the axis TA of tilt is different. As indicated by reference character XXIIIA in Figure 23, the examination head 66 is arranged at the same initial position as the first embodiment at first.

As indicated by reference characters XXIIIA and XXIIIB in Figure 23, the drive controlling unit 46 drives the XZ movement mechanism 16 and the Y movement mechanism 62 to execute a first driving process of moving, in the X, Y, and Z directions, the examination head 66 to a position where the rotation axis 64a and the rotation axis 90a coincide with a circumnutation center of the subject eye E.

The drive controlling unit 46 drives the tilt rotation mechanism 90 after completion of the first driving process to execute a second driving process of rotating the examination head 66 in the outward direction Y1 around the rotation axis 90a (the circumnutation center of the subject eye E) by the tilting angle θ (see an arrow R). With this process, as indicated by reference character XXIIIC in Figure 23, the examination head 66 is moved to the axis TA of tilt, and an optical axis O1 becomes parallel to the axis TA of tilt.

The drive controlling unit 46 then drives the XZ movement mechanism 16 and the Y movement mechanism 62 after completion of the second driving process to start a third driving process of moving the examination head 66 to an examination position along the axis TA of tilt when the examination head 66 is viewed from a one-direction side in the X direction (see an arrow YZ1). With this process, the examination head 66 is moved toward the subject eye E while keeping the tilting angle θ constant (the term "constant" as used herein is intended to include the meaning of "substantially constant"; the same applies hereinafter).

As indicated by reference character XXIIID in Figure 23, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position.

The drive controlling unit 46 switches an alignment mode of the examination head 66 to a manual alignment mode when the alignment detection unit 44 is incapable of alignment detection during a period from the start of the automatic alignment or the start of the third driving process to movement of the examination head 66 for a fixed time determined in advance or over a fixed distance.

The first driving process and the second driving process may be simultaneously executed, like the example 2-2 (see Figure 17) of the automatic alignment of the examination head 66 according to the second embodiment.

As described above, in the ophthalmic device 10 according to the fifth embodiment as well, the examination head 66 can be moved to the examination position for the subject eye E from an oblique direction (obliquely upward direction) along the axis TA of tilt at the time of the automatic alignment. As a result, the same effects as those of the second embodiment can be achieved.

Although the ophthalmic device 60A brings the examination head 66 closer to the subject eye E along the axis TA of tilt obtained by tilting the reference axis VA in the outward direction Y1 (the upward direction of the Y direction) around the subject eye E at the time of the automatic alignment of the examination head 66 in the fifth embodiment, the direction of tilt of the axis TA of tilt around the subject eye E is not particularly limited as long as the direction is a direction perpendicular to the Z direction and away from the nose N. Directions of the rotation axes 64a and 90a may be appropriately changed in accordance with the direction of tilt.

When the direction of tilt of the axis TA of tilt is fixed to the outward direction Y1 in the ophthalmic device 10A according to the fifth embodiment, the swing rotation mechanism 64 may be omitted.

### [Others]

Although alignment detection and retraction control are executed using the stereo camera 34 in the above-described first and second embodiments, the alignment detection and retraction control may be executed using an observation optical system (not illustrated) housed in the examination head 22 or 66. In this case, the stereo camera 34 and the distance sensors 70 may be omitted.

Although the stereo camera 34 is provided at the lens-barrel distal end face 28a in the first and second embodiments, the stereo camera 34 may be provided at a position other than ones at the lens-barrel distal end face 28a in the examination head 22 or 66.

Although the examination head 22 or 66 is moved to the examination position for the subject eye E along the axis TA of tilt with the tilting angle θ determined by the tilting angle determining unit 43 at the time of automatic alignment in each embodiment, the examination head 22 or 66 may be moved to the examination position for the subject eye E along the axis TA of tilt with the tilting angle θ at the time of manual alignment. Even in this case, retraction control by the retraction controlling unit 47B is automatically executed when the face distance Fd is less than the threshold determined in advance.

Although a distance between the examination head 22 or 66 and the nose N is detected as the face distance Fd under control by the detection controlling unit 47A in each embodiment, a distance between the examination head 22 or 66 and a part other than the nose N in the face of the subject may be detected.

Although a case where a displacement mechanism which displaces the examination head 22 or 66 with respect to the subject eye E includes the XZ movement mechanism 16, the Y movement mechanism 18 or 62, and the swing rotation mechanism 20 or 64 and a case where the displacement mechanism includes the tilt rotation mechanism 80 or 90 in addition to the listed components have been described as examples in the embodiments, a configuration and the type of the displacement mechanism are not particularly limited. For example, a robot arm (multijoint arm) may be used as a displacement mechanism according to the presently disclosed subject matter.

Although a multifunction machine which is a combination of a fundus camera and an optical coherence tomograph and a fundus camera alone have been described as examples of the ophthalmic device 10 in the embodiments, the presently disclosed subject matter is not limited to this. The presently disclosed subject matter can also be applied to various ophthalmic devices (including devices which perform various procedures on the subject eye E, such as a laser surgery device) which are used for examination (ocular characteristics measurement, photographing, and observation) on the subject eye E and execute alignment of various examination heads with the subject eye E, such as an optical coherence tomograph alone and an SLO device.

### {Reference Signs List}

10 ophthalmic device
12 base
14 face support
14a chin rest
14b forehead rest
16 XZ movement mechanism
18 Y movement mechanism
20 swing rotation mechanism
20a rotating shaft
22 examination head
24 fundus camera unit
26 OCT unit
28 lens-barrel
28a lens-barrel distal end face
30 objective lens
32 illumination light source
34 stereo camera
34a camera
36 vision fixation light emitting unit
37 display unit
38 manipulation unit
39 storage unit
40 control device
43 tilting angle determining unit
44 alignment detection unit
46 drive controlling unit
47A detection controlling unit
47B retraction controlling unit
48 vision fixation controlling unit
50 measurement controlling unit
52 saving controlling unit
60 ophthalmic device
62 Y movement mechanism
64 swing rotation mechanism
64a rotation axis
66 examination head
70 distance sensor
74A detection controlling unit
80, 90 tilt rotation mechanism
80a, 90a rotating shaft, rotation axis
100 objective lens
102 objective lens
104 examination head
E subject eye
F1 upper region
F2 lower region
Fd face distance
H subject
N nose
O1 optical axis
OD right eye
OS left eye
R arrow
TA axis of tilt
VA reference axis
X1 outward direction
Y1 outward direction
d1 operating distance
d2 operating distance
θ tilting angle

## Claims

1. An ophthalmic device comprising:
an examination head configured to examine a subject eye;
a displacement mechanism configured to displace the examination head with respect to the subject eye;
a drive controlling unit configured to drive the displacement mechanism to displace the examination head to an examination position for the subject eye along an axis of tilt, wherein the axis of tilt is an axis obtained by tilting, around the subject eye, a reference axis that extends along an eye direction of the subject eye in parallel with a front-back direction serving as an operating distance direction of the examination head, outward away from a subject's nose;
a distance detecting unit configured to detect a face distance which is a distance between the examination head and a face of the subject while the examination head is displaced along the axis of tilt by the displacement mechanism; and
a retraction controlling unit configured to drive the displacement mechanism to rotate the examination head in a direction of increasing the face distance around a rotation axis determined in advance when the face distance detected by the distance detecting unit is less than a threshold determined in advance.

2. The ophthalmic device according to claim 1, wherein the distance detecting unit detects, as the face distance, a distance between the examination head and the subject's nose.

3. The ophthalmic device according to claim 1, wherein
the displacement mechanism includes
a movement mechanism configured to move the examination head in the front-back direction, a left-right direction, and an up-down direction with respect to the subject eye, and
a rotation mechanism configured to rotate the examination head around the rotation axis, and
the retraction controlling unit executes first retraction control that drives the rotation mechanism to rotate the examination head in the direction of increasing the face distance and second retraction control that stops driving of the movement mechanism or drives the movement mechanism to retract the examination head in a direction away from the face.

4. The ophthalmic device according to any one of claims 1 to 3, wherein
the displacement mechanism includes
a movement mechanism configured to move the examination head in the front-back direction, a left-right direction, and an up-down direction with respect to the subject eye, and
a rotation mechanism configured to rotate the examination head around the rotation axis,
the examination head includes an objective lens,
the rotation mechanism rotates the examination head around the objective lens, and
the movement mechanism integrally moves the examination head and the rotation axis in the front-back direction, the left-right direction, and the up-down direction.

5. The ophthalmic device according to claim 4, wherein
the rotation axis is parallel to the up-down direction, and
the outward direction is parallel to the left-right direction.

6. The ophthalmic device according to claim 4, wherein
the rotation axis is perpendicular to the up-down direction, and
the outward direction is an upward direction of the up-down direction.

7. The ophthalmic device according to any one of claims 1 to 3, wherein
the displacement mechanism includes
a movement mechanism configured to move the examination head in the front-back direction, a left-right direction, and an up-down direction with respect to the subject eye, and
a rotation mechanism configured to rotate the examination head around the rotation axis,
the examination head includes an objective lens,
the rotation axis is located on a front side in the front-back direction which is closer to the subject eye than the objective lens, and
the movement mechanism integrally moves the examination head and the rotation axis in the front-back direction, the left-right direction, and the up-down direction.

8. The ophthalmic device according to claim 7, wherein
the rotation axis is parallel to the up-down direction, and
the outward direction is parallel to the left-right direction.

9. The ophthalmic device according to claim 7, wherein
the rotation axis is perpendicular to the up-down direction, and
the outward direction is an upward direction of the up-down direction.

10. The ophthalmic device according to any one of claims 1 to 3, wherein
the distance detecting unit includes
a plurality of cameras provided at the examination head, and
a detection controlling unit configured to continuously execute photographing control that causes the plurality of cameras to photograph the face from a plurality of directions different from each other and computation of the face distance based on respective photographed images of the face photographed by the plurality of cameras while the examination head is displaced along the axis of tilt by the displacement mechanism.

11. The ophthalmic device according to claim 2, wherein
the distance detecting unit includes
a plurality of cameras provided at the examination head,
a prior photographing controlling unit configured to cause one particular camera capable of photographing the nose among the plurality of cameras to photograph the nose at an initial position for the examination head, and
a detection controlling unit configured to continuously execute photographing control that causes the particular camera to photograph the nose and a computation process of comparing a photographed image of the nose newly photographed by the particular camera with a photographed image of the nose photographed at the initial position to compute the face distance while the examination head is displaced along the axis of tilt by the displacement mechanism.

12. The ophthalmic device according to any one of claims 1 to 3, wherein
the distance detecting unit includes
a non-contact distance sensor provided at the examination head, and
a detection controlling unit configured to continuously execute computation of the face distance based on a detection signal output from the distance sensor while the examination head is displaced along the axis of tilt by the displacement mechanism.

13. A method for operating an ophthalmic device comprising
an examination head configured to examine a subject eye, and
a displacement mechanism configured to displace the examination head with respect to the subject eye, the method comprising:
a drive controlling step of driving the displacement mechanism to displace the examination head to an examination position for the subject eye along an axis of tilt, wherein the axis of tilt is an axis obtained by tilting, around the subject eye, a reference axis that extends along an eye direction of the subject eye in parallel with a front-back direction serving as an operating distance direction of the examination head, outward away from a subject's nose;
a distance detecting step of detecting a face distance which is a distance between the examination head and a face of the subject while the examination head is displaced along the axis of tilt by the displacement mechanism; and
a retraction controlling step of driving the displacement mechanism to rotate the examination head in a direction of increasing the face distance around a rotation axis determined in advance when the face distance detected in the distance detecting step is less than a threshold determined in advance.
